# EUROPEAN PATENT APPLICATION

(11) **EP 4 349 850 A1**
(43) Date of publication of application: **10.04.2024**
(21) Application number: 22813883.0
(22) Date of filing: 31.03.2022
(51) Int. Cl.: C07K 7/00, C12M 3/00, C12N 5/07, C12N 5/071, C12N 5/0735, C12N 5/095, C07K 14/78

(54) **CELL CULTURE SUPPLEMENT FOR PRODUCING STEM CELLS, AND METHOD FOR PRODUCING STEM CELLS**

(30) Priority: 31.05.2021 JP 2021091262
(71) Applicant: Opticell, Inc., Yokohama-shi, Kanagawa 231-0062 (JP)
(72) Inventor: OKAMOTO, Tasuku, Kamakura-shi, Kanagawa 248-0006 (JP)
(74) Representative: Hoffmann Eitle
(86) International application number: PCT/JP2022/016934
(87) International publication number: WO 2022/254961

(57) **Abstract**

Provided are a cell culture supplement for producing stem cells and a method for producing stem cells. The cell culture supplement is formed of an aqueous solution of a polypeptide represented by the following formula (1): (Pro-Hyp-Gly)n (1) wherein Hyp represents hydroxyproline, and n represents an integer of 2 to 1000.

## Description

### Technical Field

### Cross-Reference to Related Patent Applications

The present application claims priority to Japanese patent application No. 2021-091262, filed on May 31, 2021, the entire disclosure of which is hereby incorporated by reference in its entirety. The present invention relates to a cell culture supplement for producing a stem cell and a method for producing a stem cell by using the cell culture supplement.

### Background Art

Currently, two main techniques are used to produce induced pluripotent stem cells from somatic cells. The first technique concerns iPS cells, induced pluripotent stem cells created by Dr. Shinya Yamanaka. This technique uses a viral vector to overexpress the transcription factor proteins of OCT4, SOX2, KLF4, and c-MYC in fibroblasts, and reprogram somatic cells (NPL 1). The second technique is compound-induced somatic cell direct reprogramming, first reported by Maekawa et al. (NPL 2, 3, and 4). Although iPS cell technology has already been put into practical use in the field of regenerative medicine and in applications of developed drugs in diseased organ models, avoiding the risk of immunorejection or tumor formation due to gene mutation is a major issue. Additionally, reducing the time and cost required to establish iPS cells and reprogram the cells into target organ cells is an important issue in medicine. Compound-induced direct reprogramming technology shows promise in application in individualized regenerative medicine because the technique can achieve organ regeneration using only a patient's own somatic cells and compound cocktails without the risk of tumor formation caused by gene mutation. However, at present, the organ cell types that can be produced by this technology are limited to nerve cells and adipocytes, and the development of production methods is awaited.

Recent research has revealed that cancer originates from a very small number of cells, called "cancer stem cells," and that tumors form as a heterogeneous cell population in terms of proliferative capacity and differentiation functionality (NPL 5). Like pluripotent stem cells in healthy living tissue, cancer stem cells have an ability to self-renew and an ability to differentiate into and produce a variety of cells. Although many cancer cells can be eliminated in cancer treatment by anticancer drugs, radiation, cancer immunotherapy, or surgery, recurrence and metastasis are believed to occur when a very small amount of cancer stem cells highly resistant to treatment remain. Thus, the development of new drugs, therapies, and diagnostic techniques targeting cancer stem cells is an important issue.

In order to develop new drugs, therapies, and diagnostic techniques targeting cancer stem cells, it is essential to stably produce a large amount of cancer stem cells that serve as models. For application in high-throughput screening, pharmacological testing, and safety studies, the production of cancer stem cells is essential. However, a major technical bottleneck has been the fact that the production of cancer stem cells must rely on methods to isolate a very small amount of cancer stem cells detected in specimens of cancer patients.

### Citation List

### Non-patent Literature

NPL 1: Takahashi K, Yamanaka S. Induction of pluripotent stem cells from mouse embryonic and adult fibroblast cultures by defined factors. Cell 2006; 126: 663-676.
NPL 2: Maekawa M, Yamaguchi K, Nakamura T, Shibukawa R, Kodanaka I, Ichisaka T, et al. Direct reprogramming of somatic cells is promoted by maternal transcription factor Glis1. Nature 2011; 474: 225-229
NPL 3: Yang Z, Ting Z, Hongkui D, et al. A XEN-like state bridges somatic cells to Pluripotency during chemical reprogramming. Cell 2015; 163:1678-1691.
NPL 4: Takeda Y, Harada Y, Yoshikawa T, Dai P. Chemical compound-based direct reprogramming for future clinical application. Bioscience Reports 2018; 38: 1-19.
NPL 5: Reya T, Morrison SJ, Clarke MF, et al. Stem cells, cancer, and cancer stem cells, Nature 2001; 414: 105-11.

### Summary of Invention

### Technical Problem

The technique using induced pluripotent stem cells (iPS cells) forces the expression of transcription factor genes such as SOX2, OCT4, KLF4, and c-MYC in somatic cells such as fibroblasts by using a viral vector, thus posing a risk of forming tumor by incorporating foreign genes into the chromosomes of iPS cells. Additionally, it takes six months to a year and requires significant cost to initialize fibroblasts and culture a large amount of iPS cells, then induce the differentiation of iPS cells into target organ cells, and culture the cells in quantities needed for transplantation.

Direct reprogramming, in which multiple cocktails of chemically synthesized substances are added to a culture fluid, is thought to be able to produce pluripotent stem cells directly from somatic cells in about 60 days of culture. Additionally, due to the use of small-molecule compounds, direct reprogramming involves no risk of incorporating foreign genes into chromosomes and forming tumor. In the hope of avoiding immunorejection risk due to the use of autologous fibroblasts from a patient, direct reprogramming is also expected to be put into practical use in individualized regenerative medicine. However, the organ cell types that can be produced by this technique are still limited to nerve cells and adipocytes. This technique has not yet had practical application in the medical and drug discovery fields.

The presence of cancer stem cells is known to worsen the prognosis of radiotherapy and chemotherapy. However, the method for isolating cancer stem cells has not yet been established, and medical studies are needed. There have been attempts such as a method of extracting a small number of cells from a specimen of a cancer patient, transplanting the cells into immunodeficient NOD mice to prepare tumor mass (PDOX), and isolating cells from the tumor mass by using cell sorting or a culture method by adding multiple recombinant protein factors, such as bFGF, EGF, and PDGF, or hormones. However, a stable mass-production method has not yet been established. Cancer stem cells are essential for high-throughput screening, drug efficacy evaluation, and safety assessment of drug candidates.

An object of the present invention is to provide a novel method for producing stem cells derived from somatic cells.

### Solution to Problem

To achieve the object, the inventor conducted extensive research and found that when cell adhesion proliferation was examined with a polypeptide composed only of a repeating sequence of Pro-Hyp-Gly, which is an amino acid sequence forming type I collagen, the cells did not adhere to a container coated with the polypeptide, contrary to expectation, even though the polypeptide was part of the amino acid sequence of collagen, and that the cells aggregated while floating and formed spheroids. The inventor further found that the use of an aqueous solution of the polypeptide as a cell culture supplement initializes (reprograms) somatic cells such as healthy human fibroblasts or mammary gland epithelial cells to enable the production of pluripotent stem cells with a differentiation ability. The inventor also found that cancer stem cells can be produced from a common cultured cancer cell line (cells with no stem cell markers).

The present invention was completed based on these findings and includes the following subject matter falling within a wide range of aspects.
Item 1. A cell culture supplement comprising a polypeptide represented by the following formula (1):

   (Pro-Hyp-Gly)n (1)

   wherein
   Hyp represents hydroxyproline, and
   n represents an integer of 2 to 1000.
Item 2. The cell culture supplement according to Item 1, which is for use in the production of a stem cell.
Item 3. The cell culture supplement according to Item 2, wherein the stem cell is a pluripotent cell or a cancer stem cell.
Item 4. The cell culture supplement according to Item 1, which is for use in the formation of a spheroid.
Item 5. A spheroid-forming agent comprising a polypeptide represented by the following formula (1):

   (Pro-Hyp-Gly)n (1)

   wherein
   Hyp represents hydroxyproline, and
   n represents an integer of 2 to 1000.
Item 6. A cell culture substrate having a culture surface treated with the cell culture supplement of any one of Items 1 to 4 or the spheroid-forming agent of Item 5.
Item 7. A cell culture system comprising
   the cell culture supplement of any one of Items 1 to 4 or the spheroid-forming agent of Item 5, and
   a cultured cell.
Item 8. A method for producing a stem cell, comprising
   culturing a cell by using the cell culture supplement of any one Items 1 to 4 or the spheroid-forming agent of Item 5.
Item 9. The method according to Item 8, wherein the stem cell expresses at least one undifferentiated stem cell marker gene selected from the group consisting of SOX2, OCT4, and NANOG.
Item 10. The method according to Item 8 or 9, wherein the stem cell is a pluripotent stem cell or a cancer stem cell.
Item 11. A method for producing a tissue, using a stem cell obtained by the method of any one of Items 8 to 10.

### Advantageous Effects of Invention

The present invention provides a cell culture supplement for producing stem cells. The novel cell culture supplement of the present invention also provides a method for producing somatic cell-derived stem cells. The stem cell production method of the present invention allows the polypeptide of the cell culture supplement to exert a receptor-ligand interaction, like scaffolds or adhesion factors necessary for cell culture, and even to act as a signaling factor in a similar way as that of cytokines, chemokines, hormones, etc., to produce stem cells in a simple and efficient manner. This enables simple production of autologous stem cells of a patient with a disease and achieves individualized regenerative medicine, cell therapies, and transplant therapies without the risk of forming tumor by gene mutation, as seen in iPS cells, and avoiding immunorejection.

Additionally, the use of the cell culture supplement of the present invention enables the production of cancer stem cells that express a cancer stem cell marker gene and an undifferentiated stem cell marker gene.

### Brief Description of Drawings

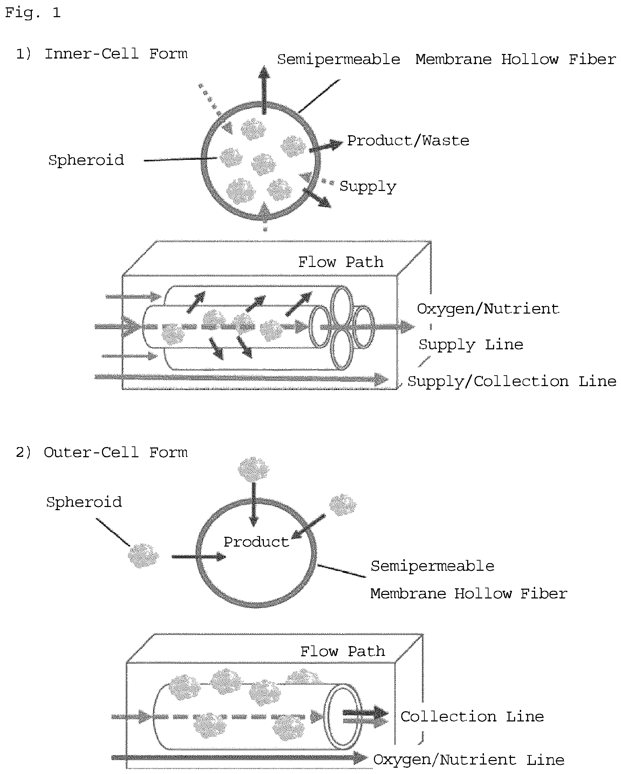

Fig. 1 shows an example of the cell culture supplement used in a cell culture substrate (semipermeable membrane, hollow fiber). A semipermeable membrane, hollow fiber, or microfluidic device. The cell culture supplement of the present invention is applied to the inside of a semipermeable membrane or a hollow fiber, or added to a medium to use the resultant as a microfluidic device.

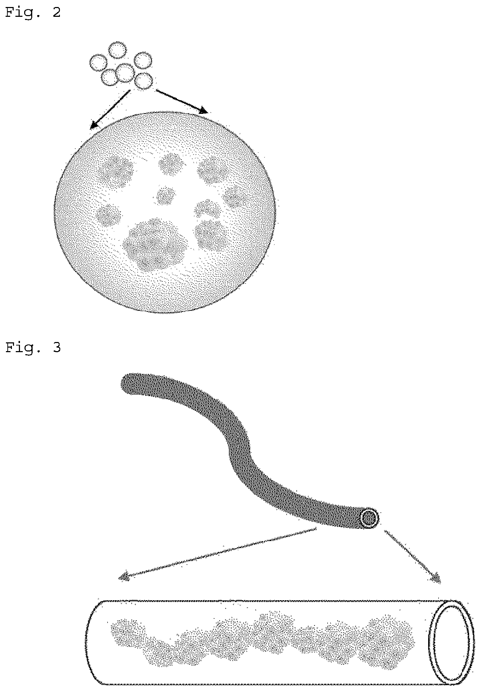

Fig. 2 shows an example of the cell culture supplement used in a cell culture substrate (capsule). A capsule formed of the cell culture supplement itself or a capsule using a biodegradable substrate. The cell culture supplement of the present invention serves as a gelled solid substrate, in which cells are embedded to form spheroids.
Fig. 3 shows an example of the cell culture supplement used in a cell culture substrate (fibrous material, semipermeable membrane, hollow fiber). The cell culture supplement of the present invention is applied to the inside of a biodegradable substrate, semipermeable membrane, hollow fiber, or fibrous material, or added to a medium, in which spheroids are placed and cultured.

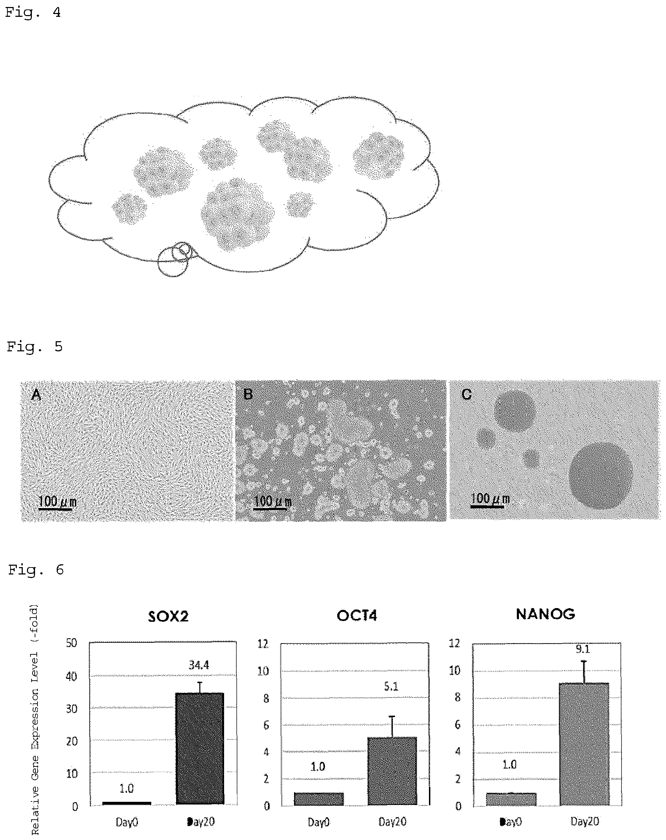

Fig. 4 shows an example of the cell culture supplement used in a cell culture substrate (a microcarrier, film, or embedding carrier based on a fibrous material). The cell culture supplement of the present invention is applied to the inside of a reticulated, semipermeable, fibrous, and/or flexible film, in which spheroids are rolled, sandwiched, or wrapped and cultured.
Fig. 5 shows two-dimensional adhesion culture and three-dimensional spheroid culture of healthy human neonatal fibroblasts. (A: planar two-dimensional culture (day 2 in culture). In a culture vessel made of PS (flat bottom) not treated with the cell culture supplement, fibroblasts adhered to the bottom and grew. B: three-dimensional culture (day 2 in culture). In a culture vessel made of PS (flat bottom) treated with the cell culture supplement, fibroblasts aggregated to form spheroids on day 2 from the start of culture. C: three-dimensional culture (day 20 in culture). In a culture vessel made of PS (flat bottom) treated with the cell culture supplement, fibroblasts formed spherical spheroids.) Healthy human neonatal fibroblasts cultured in a culture vessel coated with the cell culture supplement (polystyrene; made of PS, 6-well plate, flat bottom) self-assembled over time to aggregate and form spheroids, and grew during day 2 to day 20.
Fig. 6 shows the expression of undifferentiated stem cell marker genes in three-dimensional spheroids of healthy human neonatal fibroblasts. The cell culture supplement was applied to a culture vessel (made of PS, 6-well plate, flat bottom), and healthy human neonatal fibroblasts were cultured for 20 days to form spheroids. This resulted in increased expression levels of mRNA of the three representative undifferentiated stem cell marker genes (SOX2, OCT4, NANOG), and detection of the characteristics of stem cell induction (reprogramming).

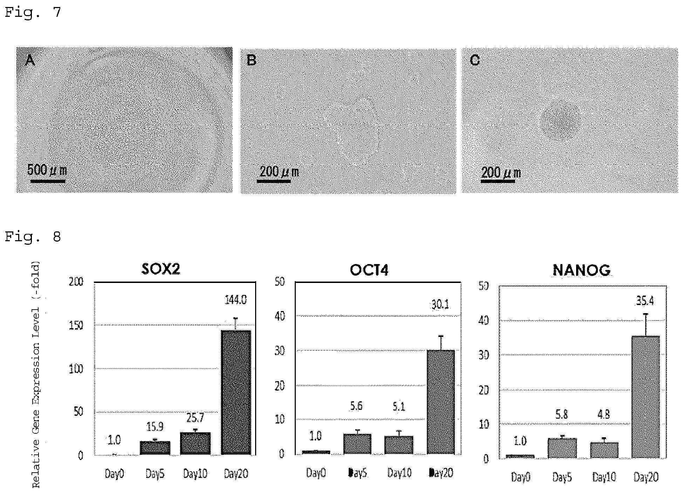

Fig. 7 shows three-dimensional spheroid culture of healthy human neonatal fibroblasts in 96-well plates. (A: 96-well (round bottom) culture (day 0). Fibroblasts (40,000 cells) were seeded in a PS-made 96-well culture vessel (round bottom) treated with the cell culture supplement. B: 96-well (round bottom) culture (day 2). The seeded fibroblasts began to aggregate and form spheroids. C: 96-well (round bottom) culture (day 20). After day 6, a single spheroid formed and remained in good condition after day 20 from the start of culture.) When the cell culture supplement was applied to a round-bottom culture vessel (made of PS, 96-well microplate), and human neonatal fibroblasts were spheroid-cultured, the cells aggregated over time to form spheroids and grew during day 2 to day 20.
Fig. 8 shows changes over time in the expression of undifferentiated stem cell marker genes in three-dimensional spheroids of healthy human neonatal fibroblasts. When the cell culture supplement was applied to a round-bottom culture vessel (PS, 96-well microplate), and healthy human neonatal fibroblasts were spheroid-cultured, the expression levels of mRNA of the three undifferentiated stem cell marker genes (SOX2, OCT4, NANOG) increased over time and reached their highest value on day 20 from the start of culture. Stem cells were found to be induced (directly reprogrammed) without gene transfer to fibroblasts (somatic cells) (the Yamanaka method for creating iPS cells).

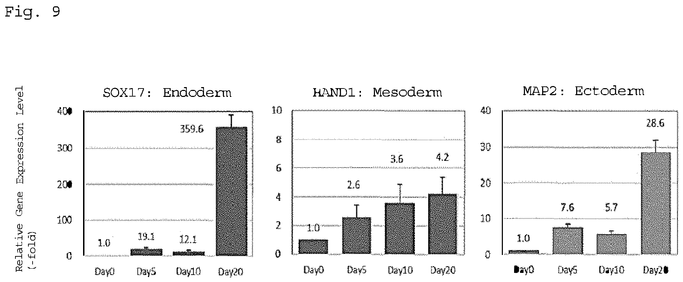

Fig. 9: Induction of differentiation into healthy human neonatal fibroblast-derived stem cells: expression of genes associated with differentiation into three germ layers. When healthy human neonatal fibroblasts were spheroid-cultured in a culture vessel coated with the cell culture supplement, the expression levels of mRNA of marker genes for differentiation into primordial three germ layers (SOX17; endoderm, GATA6; mesoderm, MAP2; ectoderm) increased over time and reached their highest levels on day 20. Stem cells were found to be induced to differentiate into three germ layers in the early stage of differentiation into various organs.

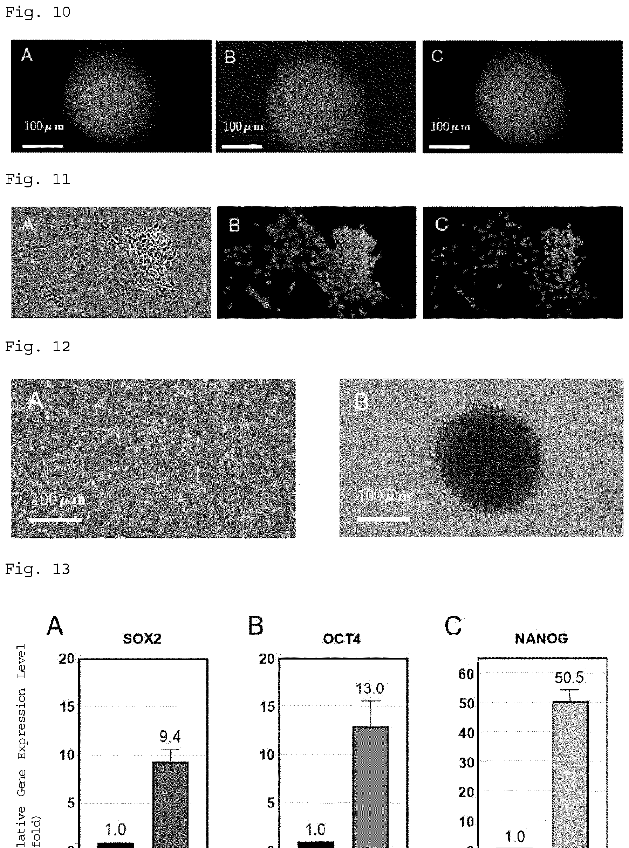

Fig. 10 shows the expression of undifferentiated stem cell marker proteins in spheroids of healthy human fibroblasts. Immunostaining using fluorescent dye-labeled antibodies was able to detect stem cell marker proteins SOX2, OCT4, and NANOG, indicating the presence of stem cells in the cells forming spheroids. A: fluorescence detected by NL557-labeled anti-SOX2 antibody. B: fluorescence detected by NL637-labeled anti-OCT4 antibody. C: fluorescence detected by NL493-labeled anti-NANOG antibody.
Fig. 11 shows NANOG protein-positive stem cells present within spheroids. After 20 days of culture, spheroids were dissociated with an Accutase enzyme reagent and immunostained with a fluorescent dye-labeled anti-NANOG antibody. In some spheroid-forming cells, nuclear NANOG-positive stem cells were found to be induced. A: some of the cells dissociated from spheroids, bright field image. B: NANOG antibody-positive cells (the same cell population as A), NANOG protein-positive stem cells were detected in the cell nuclei. C: Staining of nuclei with DAPI (the same cell population as in A), nuclear DNA stained in blue showing the shape of nuclei (control image).
Fig. 12 shows a two-dimensional culture and a three-dimensional spheroid culture of adult skin fibroblasts. Adult skin fibroblasts, which are easy to collect from the human body with minimum invasion, were used to demonstrate the feasibility of spheroid culture by using the cell culture supplement of the present invention. A: planar two-dimensional culture; adult skin fibroblasts adhered to the bottom of a PS culture vessel and grew. B: three-dimensional culture (day 20 in culture); adult skin fibroblasts forming a spheroid in a 96-well culture vessel treated with the cell culture supplement.
Fig. 13 shows the analysis of the expression of undifferentiated stem cell marker genes in three-dimensional spheroid culture of adult skin fibroblasts. Stem cells were shown to be induced by the spheroid culture method using the cell culture supplement from skin-derived fibroblasts, which are easy to collect with minimum invasion (essential for the implementation of individualized regenerative therapy). A: SOX2. B: OCT4. C: NANOG.

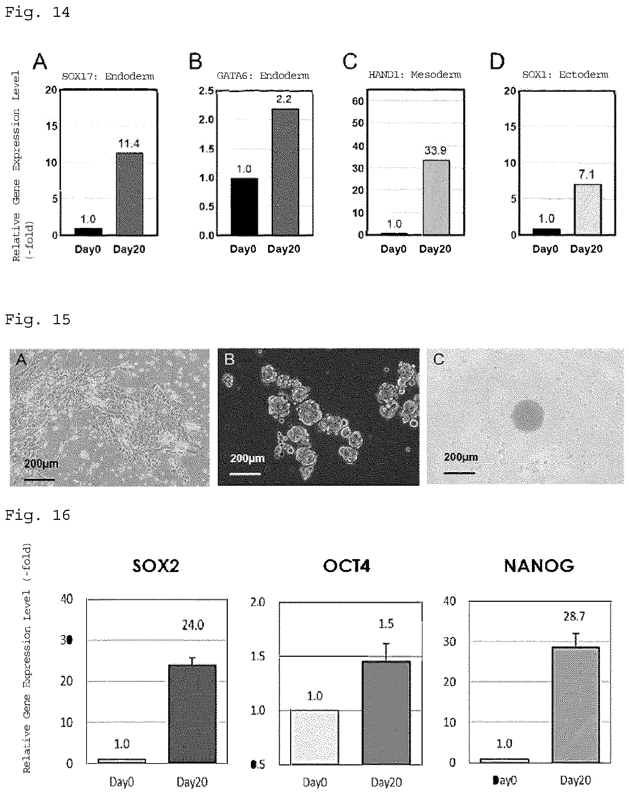

Fig. 14 shows the analysis of the expression of genes associated with differentiation of three germ layers in three-dimensional spheroid culture of adult skin fibroblasts. Stem cells were shown to be induced by the spheroid culture method using the cell culture supplement from skin-derived fibroblasts, which are easy to collect with minimum invasion (essential for the implementation of individualized regenerative therapy). A: SOX17. B: GATA6. C: HAND1. D: SOX1.
Fig. 15 shows two-dimensional adhesion culture and three-dimensional spheroid culture of healthy human adult mammary gland epithelial cells. (A: two-dimensional culture (day 2 in culture). In a culture vessel made of PS (flat bottom) without the cell culture supplement, mammary gland epithelial cells adhered to the bottom and grew. B: three-dimensional culture (day 2 in culture). Mammary gland epithelial cells formed spheroids in a culture vessel made of PS (flat bottom) treated with the cell culture supplement. C: three-dimensional culture (day 20 in culture). A spheroid of mammary gland epithelial cells on day 20 in culture in a 96-well culture vessel (round bottom) treated with the cell culture supplement.) When healthy human adult mammary gland epithelial cells were cultured in a PS culture vessel (flat bottom or round bottom) treated with the cell culture supplement, the cells aggregated over time to form spheroids and grew during day 2 to day 20. Feasibility was demonstrated with both adult-derived cells and neonatal-derived cells, as well as with a wide range of cell types, including epithelial cells and fibroblasts.
Fig. 16 shows the detection of undifferentiated stem cell marker genes in three-dimensional spheroid culture of healthy human adult mammary gland epithelial cells. When healthy human adult mammary gland epithelial cells were cultured in a culture vessel coated with the cell culture supplement to form spheroids, the expression levels of mRNA of the three representative undifferentiated stem cell markers (SOX2, OCT4, NANOG) increased and reached their highest levels on day 20 from the start of culture. Stem cells were found to be induced (directly reprogrammed) without gene transfer to epithelial cells (somatic cells).

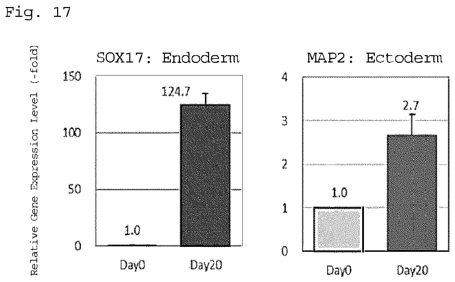

Fig. 17: Induction of differentiation into healthy human adult mammary gland epithelial cell-derived stem cells: expression of genes associated with three germ layers. When healthy human adult mammary gland epithelial cells were cultured for 20 days in a culture vessel coated with the cell culture supplement to form spheroids, the expression levels of mRNA of SOX17 (endoderm) and MAP2 (ectoderm), among primordial three germ layer marker genes, reached their highest levels on day 20. However, the expression level of FOXA2 (endoderm) was decreased, and GATA4 (mesoderm) was undetectable. Stem cells were found to be induced to differentiate into three germ layers in the early stage of differentiation into various organs.

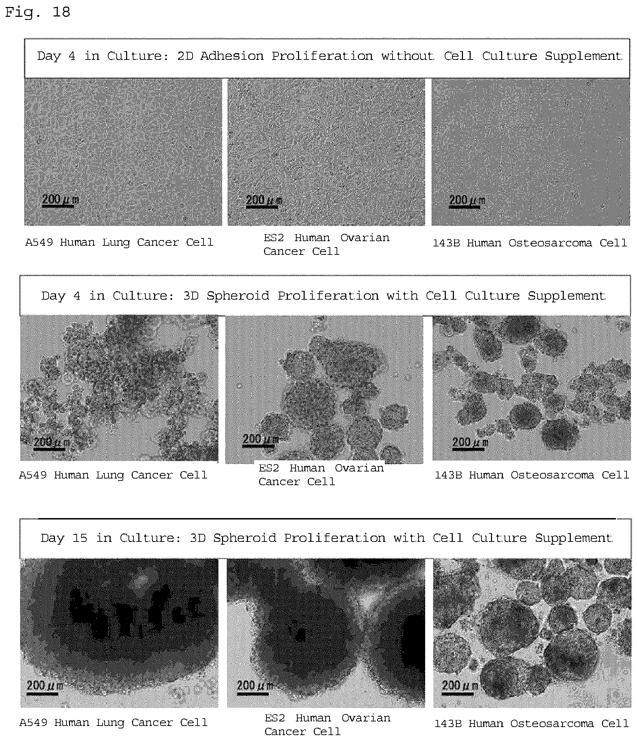

Fig. 18 shows two-dimensional adhesion proliferation and three-dimensional spheroid proliferation of cancer cells with and without the cell culture supplement. When three types of cancer cells (A549 cells; human lung cancer, ES2 cells; human ovarian cancer, 143B; human osteosarcoma) were cultured in a culture vessel (polystyrene; made of PS, 6-well plate, flat bottom) coated with the cell culture supplement, the cells aggregated over time to form spheroids and grew until day 15.

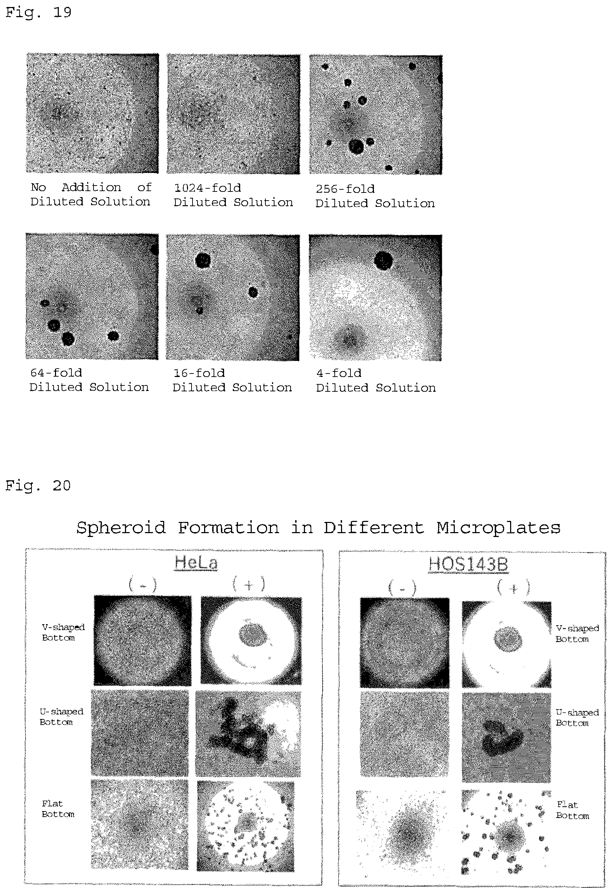

Fig. 19 shows the relationship between the concentration of the cell culture supplement and spheroids.
Fig. 20 shows the results of spheroid formation in various microplates.

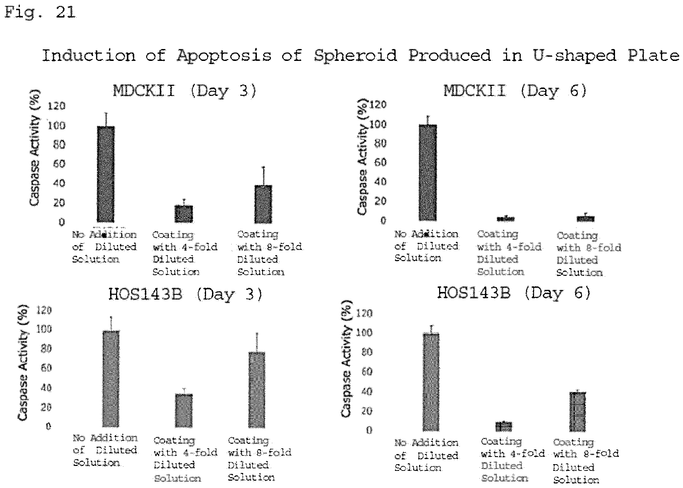

Fig. 21 shows the quantitative results of induction of apoptosis in spheroids prepared in a U-shaped plate.

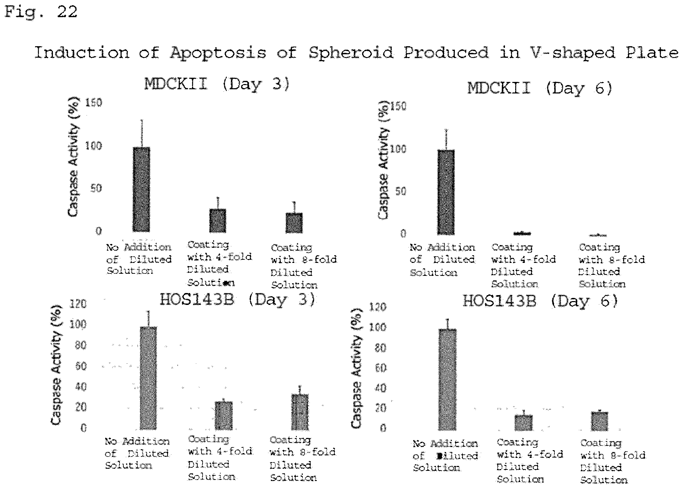

Fig. 22 shows the quantitative results of induction of apoptosis in spheroids prepared in a V-shaped plate.

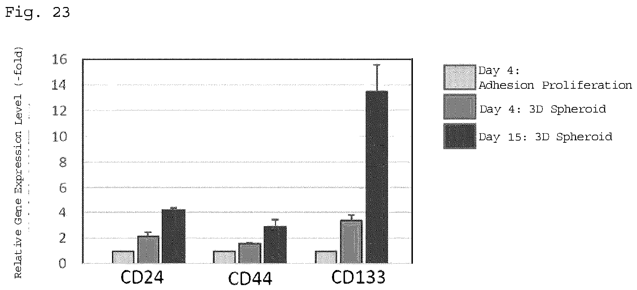

Fig. 23 shows the expression levels of cancer stem cell marker genes in A549 human lung cancer cells. When A549 human lung cancer cells were spheroid-cultured in a culture vessel coated with the cell culture supplement, the expression levels of mRNA of three cancer stem cell marker genes (CD24, CD44, and CD133) increased over time and reached their highest levels on day 15 from the start of culture. Cells with the characteristics of lung cancer stem cells were found to be producible.

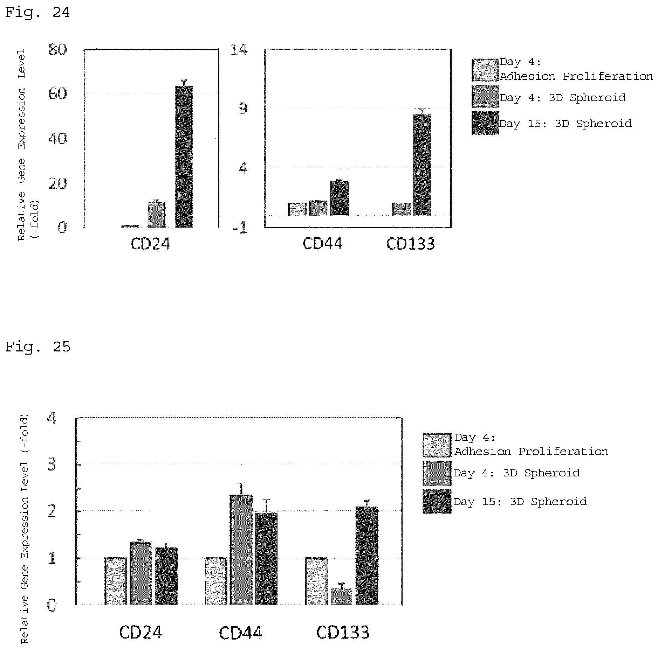

Fig. 24 shows the expression levels of cancer stem cell marker genes in ES2 human ovarian cancer cells. When ES2 human ovarian cancer cells were spheroid-cultured in a culture vessel coated with the cell culture supplement, the expression levels of mRNA of three cancer stem cell marker genes (CD24, CD44, and CD133) increased over time and reached their highest levels on day 15 from the start of culture. Cells with the characteristics of ovarian cancer stem cells were found to be producible.
Fig. 25 show the expression levels of cancer stem cell marker genes in 143B human osteosarcoma cells. When 143B human osteosarcoma cells were spheroid-cultured in a culture vessel coated with the cell culture supplement, the expression of mRNA of two genes CD44 and CD133, among the three cancer stem cell marker genes, increased over time, and reached their highest levels on day 15 from the start of culture. CD24 did not increase significantly. Cells with the characteristics of osteosarcoma stem cells were found to be producible.

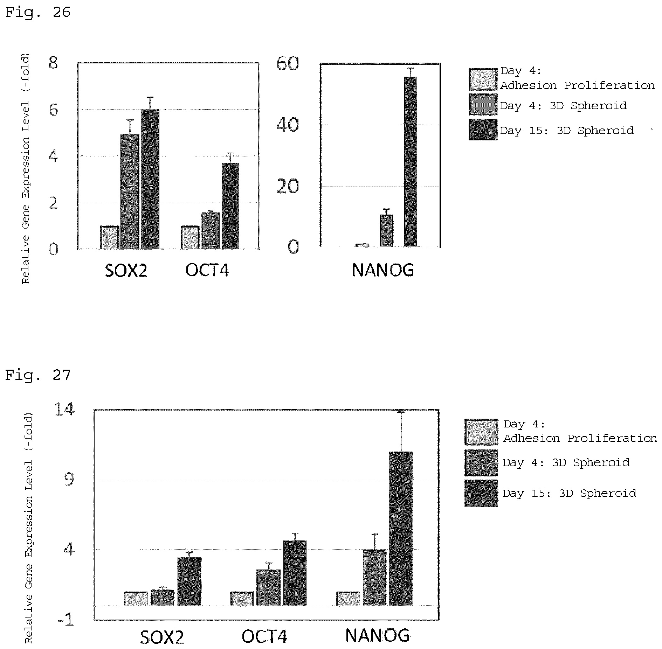

Fig. 26 shows the detection of genes in three-dimensional spheroids of A549 human lung cancer cells. When A549 human lung cancer cells were spheroid-cultured in a culture vessel coated with the cell culture supplement, the expression levels of mRNA of the three representative undifferentiated stem cell marker genes (SOX2, OCT4, NANOG) increased over time and reached their highest levels on day 15 from the start of culture.
Fig. 27 shows the detection of genes in three-dimensional spheroids of ES2 human ovarian cancer cells. When ES2 human ovarian cancer cells were spheroid-cultured in a culture vessel coated with the cell culture supplement, the expression levels of mRNA of three undifferentiated stem cell marker genes (SOX2, OCT4, NANOG) increased over time and reached their highest levels on day 15 from the start of culture.

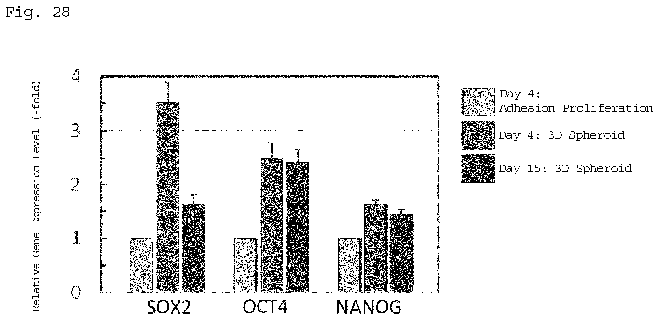

Fig. 28 shows the detection of undifferentiated stem cell marker genes in three-dimensional spheroids of 143B human osteosarcoma cells. When 143B human osteosarcoma cells were spheroid-cultured in a culture vessel coated with the cell culture supplement, the expression levels of mRNA of three undifferentiated stem cell marker genes (SOX2, OCT4, NANOG) increased during day 4 to day 15 from the start of culture.

### Description of Embodiments

In the present specification, the singular nouns (with an article such as a, an, or the) include the concepts of singular and plural nouns, unless otherwise explicitly indicated in the specification or the context clearly conflicts.

### Cell Culture Supplement

The cell culture supplement of the present invention contains a polypeptide represented by the following formula (1):

(Pro-Hyp-Gly)ₙ (1)

wherein Hyp represents hydroxyproline, and n represents an integer of 2 to 1000.

In the formula, n represents an integer of 2 to 1000, and n is preferably an integer of 10 to 1000, and more preferably an integer of 500 to 1000.

The cell culture supplement of the present invention can be used in the production of stem cells. The use of the cell culture supplement of the present invention in cell culture allows cultured cells to form spheroids, enabling the production of stem cells simply and efficiently.

In the present specification, a spheroid (also referred to as a "mass of cells") encompasses multiple cells that are three-dimensionally assembled and adhered to each other.

The polypeptide represented by formula (1) can be produced according to a known production method. For example, the polypeptide represented by formula (1) can be produced by condensation reaction of a peptide oligomer represented by (Pro-Hyp-Gly)ₘ (wherein m is an integer of, for example, about 1 to 10, 1 to 5, 10 to 1000, or 500 to 1000).

The cell culture supplement of the present invention preferably contains an aqueous solution of the polypeptide represented by formula (1). The aqueous solution preferably contains the polypeptide represented by formula (1) in an amount of about 0.001 to 0.5 wt% (w/v), and more preferably about 0.01 to 0.25 wt% (w/v). The cell culture supplement of the present invention may be an aqueous solution substantially containing no component other than the polypeptide represented by formula (1), but is not limited thereto.

The aqueous solution of the polypeptide represented by formula (1) for use may be a commercial product, such as PURECOLLA (JNC Corporation).

### Use in Surface Treatment Agent or Additive

In an embodiment, the cell culture supplement of the present invention can be used in treating a culture surface of a cell culture substrate.

Such a cell culture substrate is not particularly limited, and examples include dishes, petri dishes, microwell plates (with a flat bottom, U-shaped bottom, V-shaped bottom, etc.), tubes, films, fibrous materials, semipermeable membranes, hollow membranes, hollow fibers, capsules, and microcarriers. The cell culture substrate for use can be made from, for example, glass, metal, plastics, such as polystyrene (PS), polypropylene (PP), polycarbonate (PC), polyethylene terephthalate (PET), polytetrafluoroethylene (PTFE), polyvinylidene fluoride (PVDF), cycloolefins (COP), poly methyl methacrylate (PMMA), hydrophilic fluorinated resins, polylactic acid (PLA), polybutylene succinate (PBS), or polyhydroxybutyrate/hydroxyhexanoate (PHBH), a cellulose film, a carbon nanotube, cellulose nanofibers, etc.

Figs. 1 to 4 show examples of the cell culture supplement used in a cell culture substrate; however, embodiments are not limited to these examples.

Fig. 1 shows an example of application of the cell culture supplement in a semipermeable membrane or a hollow fiber. As shown in Fig. 1, the inside of a semipermeable membrane or a hollow fiber can be coated with the cell culture supplement of the present invention, or the cell culture supplement can be added to a medium, and such a membrane, fiber, or medium can be used as a microfluidic device. The microfluidic device is expected to have application, for example, in the areas of (i) reproduction of functions of organs, such as kidney or liver, (ii) (carry-type) medical devices for extracorporeal circulation organs, such as kidney or liver, (iii) production of recombinant proteins, such as monoclonal antibodies, and (iv) drug discovery model/pharmacological evaluation, and toxicity evaluation.

Fig. 2 shows an example of application of the cell culture supplement in a capsule. As shown in Fig. 2, the cell culture supplement of the present invention can be used as a gelled solid substrate, in which cells can be embedded to form spheroids. Such a capsule is expected to have application, for example, in the areas of (i) organs for regenerative medicine: carriers for transplanting stem cells or differentiated organoids into the body, (ii) transfusion of hematopoietic stem cells, and (iii) production of artificial muscles.

Fig. 3 shows an example of application of the cell culture supplement in a fibrous material, a semipermeable membrane, or a hollow fiber. As shown in Fig. 3, the lumen of a biodegradable substrate, a semipermeable membrane, a hollow fiber, or a fibrous material can be coated with the cell culture supplement of the present invention, or the cell culture supplement can be added to a medium, in which spheroids can be placed and cultured. The fibrous material, semipermeable membrane, and hollow fiber are expected to have application, for example, in the areas of (i) organs for regenerative medicine: transplantation and regeneration of thin and long organs, such as blood vessels, nerves, intestinal tracts, and renal tubules, and (ii) sensor circuits for electric potential, light, odor components, taste components, etc., in nerves.

Fig. 4 shows an example of application of the cell culture supplement of the present invention in a microcarrier. As shown in Fig. 4, the inside of a reticulated, semipermeable, fibrous, or flexible film can be coated with the cell culture supplement of the present invention, and spheroids can be rolled, sandwiched, or wrapped and then cultured in the film. Such a microcarrier is expected to have application, for example, in the area of transplantation and regeneration of pancreas, kidney, liver, bone marrow, bone, cartilage, muscle, skin, hair, etc. in regenerative medicine.

If the cell culture supplement of the present invention is used to treat the culture surface of a cell culture substrate, the cell culture substrate for use does not have to be a culture vessel with an ultra-low adhesion surface treated with hydrophilic processing (e.g., dishes, flasks, microfluidic devices, cell stack chambers, and bags). The use of the cell culture supplement of the present invention enables simple formation of spheroids without a culture vessel with an ultra-low adhesion surface and thus enables production of pluripotent stem cells and cancer stem cells.

The culture surface of a cell culture substrate refers to the surface that the cells come in contact with during culture. The culture surface can be treated partly or in its entirety. It is not precluded that surfaces other than the culture surface be treated.

The surface treatment can be any means that can immobilize (e.g., by coating) the polypeptide represented by formula (1), which is the active ingredient of the cell culture supplement of the present invention, to the culture surface of the cell culture substrate. Examples include coating, dipping, and spraying. If a cell culture substrate made of plastic is used, it is optional but preferable to perform a surface treatment such as plasma treatment first, and to perform surface treatment with the cell culture supplement of the present invention. Known additives may be mixed with the cell culture supplement of the present invention for use in surface treatment.

For use in the surface treatment of the culture surface of a cell culture substrate, the concentration of the polypeptide represented by formula (1), which is the active ingredient of the cell culture supplement of the present invention, is not limited and can be, for example, 0.001 to 0.5 wt% (w/v), and preferably 0.01 to 0.25 wt% (w/v).

When the cell culture supplement of the present invention is used in treating the surface of a cell culture substrate, the surface treatment may be followed by drying to remove water. The means of drying is not limited, and can be, for example, vacuum drying or a desiccant.

Additionally, after drying, the cell culture substrate can be sterilized. The means of sterilization is not limited, and can be, for example, ethylene oxide gas, electron beams, gamma rays, or an autoclave.

In another embodiment, the cell culture supplement of the present invention can be added to a culture medium and used.

The culture medium to which the cell culture supplement of the present invention is added can be selected from a wide range of mediums used for common cell culture, such as liquid mediums and gelled mediums. The medium may contain serum or plasma additives, such as FBS and FCS, which are animal-derived ingredients, and protein additives, such as albumin. Examples include α-MEM mediums, Neurobasal mediums, Neural Progenitor Basal mediums, NS-A mediums, BME mediums, BGJb mediums, CMRL 1066 mediums, minimum essential mediums (MEM), GMEM mediums, Eagle MEM mediums, Dulbecco's modified Eagle mediums (DMEM), Glasgow MEM mediums, Improved MEM Zinc Option mediums, IMDM mediums, Medium 199 mediums, DMEM/F12 mediums, StemPro-34SFM mediums, Ham's mediums, RPMI 1640 mediums, HTF mediums, and Fischer's mediums. Additionally, examples of xeno-free or serum-free mediums, free of animal-derived ingredients, include mTeSR mediums, Stem Fit mediums, Cellartis DEF-CS500 mediums, Cellartis MSC-CS500 mediums, NutriStem mediums, PluriSTEM Human ES/iPS mediums, fibroblast growth mediums, and mammary epithelial cell growth medium. Examples of gelled mediums include, but are not limited to, soft agar mediums, Matrigel mediums, and methylcellulose-based mediums. The mediums may also contain other known additives.

The amount of the cell culture supplement of the present invention added to a culture medium is not limited and can be, for example, 0.001 to 0.5 wt% (w/v), and preferably 0.01 to 0.25 wt% (w/v) in terms of the final concentration of the polypeptide represented by formula (1), which is the active ingredient of the cell culture supplement of the present invention.

A cell culture substrate having a surface treated with the cell culture supplement of the present invention and a culture medium to which the cell culture supplement of the present invention has been added can also be used in combination in cell culture.

### Spheroid-forming Agent

The cell culture supplement of the present invention, containing the polypeptide represented by formula (1), can be used as a spheroid-forming agent in another embodiment.

A spheroid can be formed according to a commonly used method for forming spheroids, except that cell culture is performed using a cell culture apparatus having the cell culture supplement of the present invention on the culture surface, or that cell culture is performed in a culture medium to which the cell culture supplement of the present invention has been added.

Commonly used methods for forming spheroids include a non-adhesive surface cell culture method, a hanging-drop cell culture method, use of a micromolding technique, and a rotary cell culture method.

The cells for use in forming spheroids are not limited, and examples include fibroblasts derived from skin tissue, epithelial cells derived from various organs, and primary cultured cells of somatic cells derived from various tissues, such as lung, liver, pancreas, kidney, intestine, cartilage, bone, and central nerve, and peripheral nerves. However, these primary cultured cells often lose their original functions and may evoke abnormal activation when undergo planar culture, and may even induce apoptosis, also known as anoikis (programmed cell death), which is problematic. Under such circumstances, forming spheroids of primary cultured cells with the cell culture supplement of the present invention was found to be effective in inhibiting the development of apoptosis and maintaining the physiological functions while assisting their survival and proliferation. Thus, spheroid-formed cells are expected to be able to maintain biological functionality in higher levels as compared with cells cultured in planar culture and provide a model that reflects biological functionality. It is also useful to form spheroids of an established cancer cell line. It is known that spheroids formed of cancer cells can reproduce the "malignant tumor functionality" as they existed in the patient's body, and maintain the active status of enzymes, pump receptors, etc. that lead to resistance to anticancer drugs. Therefore, cancer cell spheroids are expected to have application in drug discovery research as a cancer patient model that can reproduce pathological conditions. Additionally, in the induction of differentiation of iPS cells, spheroids are often cultured to produce embryoid bodies, which are then differentiated into various organs. In this case, it is known that spheroid formation facilitates the differentiation of iPS cells into three germ layers (endoderm, mesoderm, and ectoderm), and its major usefulness is to promote the process of inducing the differentiation into organ cells.

The medium for spheroid formation may be a serum-containing or serum-free medium. Examples of serum-containing mediums include, but are not limited to, αMEM mediums, Neurobasal mediums, Neural Progenitor Basal mediums, NS-A mediums, BME mediums, BGJb mediums, CMRL 1066 mediums, minimum essential mediums (MEM), GMEM mediums, Eagle MEM mediums, Dulbecco's Modified Eagle Mediums (DMEM), Glasgow MEM mediums, Improved MEM Zinc Option mediums, IMDM mediums, Medium 199 mediums, DMEM/F12 mediums, StemPro-34SFM mediums, Ham's mediums, RPMI 1640 mediums, HTF mediums, Fischer's mediums, and combination mediums of these. The concentration of serum (e.g., fetal bovine serum (FBS) or human serum) to be added is 2 to 20%, and preferably 5 to 10%.

The serum-free medium (SFM) refers to both a medium free of animal-derived ingredients (xeno-free) and a medium free of untreated or unpurified serum (serum-free). The serum-free medium includes a medium that contains growth factors (basic-FGF, EGF, insulin, etc.) produced according to a genetic recombination method. The serum-free medium may contain any serum substitute. Examples of serum substitutes include KnockOut serum replacements, albumin (e.g., lipid-rich albumin, albumin substitutes such as recombinant albumin, plant starch, dextrans, and protein hydrolysates), transferrins (or other iron transporters), fatty acids, insulin, collagen precursors, trace elements, 2-mercaptoethanol, 3'-thioglycerol, and their equivalents. These can be used alone or in a combination of two or more.

The medium may contain other known additives. Examples of additives include, but are not limited to, growth factors (e.g., insulin, growth hormones, EGF, FGF, TGF, BMP, Activin, Wnt, LIF, and Noggin), valproic acid, forskolin, retinoic acid, various inhibitors or activators (e.g., A83-01, DZNep, Y27632, SB202190, PD0325901, and CHIR99021), polyamines, minerals, carbohydrates (e.g., glucose), organic acids (e.g., pyruvic acid and lactic acid), amino acids (e.g., nonessential amino acids (NEAA) and L-glutamine), reducing agents (e.g., 2-mercaptoethanol), vitamins (e.g., ascorbic acid and d-biotin), steroids, antibiotics (e.g., streptomycin, and penicillin), surfactants (e.g., Tween-20 and Nonidet P-40), buffers (e.g., HEPES), and nutrient additives (e.g., B27 supplement, N2 supplement, StemPro-Nutrient Supplement, KnockOut Media, and GlutaMAX). These can be used alone or in a combination of two or more. Each additive is preferably included in a known concentration range.

The culture conditions for forming spheroids are not limited as long as intended spheroids are formed. For example, the culture temperature may be 20 to 40°C, and preferably 34 to 38°C. The culture can also be performed in a 3 to 10% CO₂ atmosphere, and preferably in a 4 to 6% CO₂ atmosphere. The culture period for forming spheroids is, for example, about 0.5 hours to 14 days, and preferably about 1 day to 4 days. Optionally, the medium may be replaced one or more times during the culture period.

The size of a spheroid is typically about 10 to 500 µm, and preferably about 50 to 200 um in diameter. It is preferred to separate spheroids that have reached a suitable size because it is difficult for nutrients to penetrate into the interior of overly large spheroids. The resulting spheroids can be disassembled by enzymatic treatment with trypsin and/or collagenase and then cultured again with the cell culture supplement of the present invention to form spheroids repeatedly.

The concentration of cells in culture is not limited as long as spheroids are formed efficiently. For example, the concentration of cells in culture is about 1 × 10³ to about 1 × 10⁷ cells/well, and preferably about 3 × 10³ to about 5 × 10⁶ cells/well.

In the method of forming spheroids using the cell culture supplement of the present invention, the polypeptide of the cell culture supplement of the present invention is thought to exert a receptor or ligand action such as a scaffold or adhesion factor necessary for cultured cells, and further act as a signaling factor similar to cytokines, chemokines, hormones, etc. to promote the self-assembly of the cells, thereby facilitating the formation of spheroids.

Another embodiment of the present invention provides a cell culture system containing the cell culture supplement of the present invention and cultured cells. The cultured cells for use are selected from the wide range of cells listed above for spheroid formation. For preparing pluripotent stem cells or cancer stem cells using the cell culture system, it is preferred that somatic cells or cancer cells are used, respectively.

Another embodiment of the present invention provides a method for producing a stem cell, including culturing cells using the cell culture supplement of the present invention.

"Stem cells" in the present invention refer to cells that express an undifferentiated stem cell marker gene. Examples include pluripotent stem cells, cancer stem cells, somatic stem cells, and mesenchymal stem cells. Of these, the cell culture supplement of the present invention is preferably used in the production of pluripotent stem cells and cancer stem cells. The following describes in detail the methods for producing pluripotent stem cells and cancer stem cells.

### Method for Producing Pluripotent Stem Cells

Another embodiment of the present invention provides a method for producing pluripotent stem cells, including culturing somatic cells by using the cell culture supplement of the present invention. Culturing somatic cells by using the cell culture supplement of the present invention allows the cultured cells to form spheroids, enabling the simple and efficient production of pluripotent stem cells.

The method for using the cell culture supplement of the present invention in the production of pluripotent stem cells includes, but is not limited to, for example, using the cell culture supplement to treat the culture surface of a cell culture substrate and/or adding the cell culture supplement to a culture medium. Preferably, somatic cells are cultured using a cell culture substrate having the culture surface treated with the cell culture supplement of the present invention. The use of the cell culture supplement for surface treatment of the culture surface of a cell culture substrate and the use by adding the cell culture supplement to a culture medium are the same as described in section "Use in Surface Treatment Agent or Additive" above.

The somatic cells for use in the production of pluripotent stem cells of the present invention can be any somatic cells derived from a mammal (humans, mice, rats, cows, pigs, monkeys, sheep, rabbits, and dogs) and can be selected from a wide range. Of these, humans, monkeys, and mice are preferred, and humans are particularly preferred. The type of somatic cells for use is not limited and can be selected from a wide range of those, such as fibroblasts, epithelial cells, cartilage cells, muscle cells, bone cells, liver cells, pancreatic cells, intestinal cells, nerve cells, bone marrow cells, and skin cells. Somatic cells derived from healthy individuals and somatic cells derived from patients with a disease can both be used in the production of pluripotent stem cells of the present invention.

The mediums for use in the production of pluripotent stem cells can be selected from a wide range of those listed in section "Spheroid-forming Agent" above.

Additionally, the method for producing pluripotent stem cells of the present invention does not require adding various adjuvants for differential proliferation, such as cell growth factors (e.g., PDGF, TGF-β, Activin, BMP, Noggin, LIF, R-Spondin, Wnt-3a, EGF, TPO, SCF, and Neuregulin), mesenchymal stem cell maintenance mediums, and retinoic acid. Using the cell culture supplement of the present invention enables the production of pluripotent stem cells from somatic cells without adding cell growth factors to the medium.

The method for producing pluripotent stem cells can be performed according to a commonly used method for forming spheroids, except for performing cell culture by using a cell culture substrate having the cell culture supplement of the present invention on its culture surface. Examples include a non-adhesive surface cell culture method, a hanging-drop cell culture method, use of a micromolding technique, and a rotary cell culture method, with a non-adhesive surface cell culture method being particularly preferred.

The culture conditions for producing pluripotent stem cells are not limited. For example, the culture temperature may be 20 to 40°C, and preferably 34 to 38°C. The culture can also be performed in a 3 to 10% CO₂ atmosphere, and preferably in a 4 to 6% CO₂ atmosphere. The culture period is, for example, about 10 to 40 days, and preferably about 15 to 30 days. Optionally, the medium may be replaced one or more times during the culture period. Optionally, the cell culture substrate may be replaced one or more times during the culture period.

The size of a spheroid in the production of pluripotent stem cells is typically about 10 to 500 µm, and preferably about 50 to 200 µm in diameter. It is preferred to separate spheroids that have reached a suitable size because it is difficult for nutrients to penetrate into the interior of overly large spheroids. The resulting spheroids can be disassembled by enzymatic treatment with trypsin and/or collagenase, and then cultured again with the cell culture supplement of the present invention to form spheroids that have the functionality of pluripotent stem cells again.

The concentration of somatic cells in culture is not limited as long as pluripotent stem cells are efficiently produced. For example, the concentration of somatic cells in culture is about 1 × 10³ to about 1 × 10⁷ cells/well, and preferably about 3 × 10³ to about 5 × 10⁶ cells/well.

In the method for producing pluripotent stem cells of the present invention, the production of pluripotent stem cells can be confirmed by identifying the expression of undifferentiated stem cell markers. Examples of undifferentiated stem cell markers include OCT4, SOX1, SOX2, SOX3, SOX18, NANOG, Klf2, Klf4, Essrb, Sall4, Tell, Tcl3, c-Myc, c-Mycn, LIN28, SSEA-1, SSEA-3, SSEA-4, TRA-1-60, TRA-1-81, AP, Fzd1-10, and TDGF-1.

Whether the pluripotent stem cells obtained by the present invention have a differentiation-inducing ability can be determined by confirming genes associated with three germ layers. Examples of genes associated with the three germ layers (endoderm, mesoderm, and ectoderm) include SOX17, FOXA2, Nestin, Flk1/KDRAFP, Cripto, Bmp4/Wnt3, GATA4, GATA6, Brachyury, HAND1, CXCR4, SOX1, Otx-2, Pax2, Pax6, and MAP2.

The pluripotent stem cells obtained by the production method of the present invention may be a population of heterogeneous cells in various differentiation states, such as those with phenotypic characteristics of staged embryonic differentiation, including ES cells, neural stem cells, mesenchymal stem cells, extraembryonic endoderm cells prepared by cell fate transformation using transcription factors (iPS cells), extraembryonic endoderm cells prepared by using compound cocktails (e.g., various cytokines, hormones, agonists, and inhibitors) (XEN-like cells), and those with characteristics of embryoid bodies obtained in floating culture of iPS cells.

The method for producing pluripotent stem cells of the present invention can produce pluripotent stem cells in a very short period of time (about 10 to 40 days) compared to the culture period of induced pluripotent stem cells (iPS cells), which is six months to one year. There is also no risk of tumor formation in transplantation, as known in induced pluripotent stem cells (iPS cells), because no foreign genes are incorporated. Additionally, immunorejection can be avoided because one's own pluripotent stem cells can simply be created from somatic cells, such as fibroblasts, which are easily harvested even from human skin tissue. Thus, if application in transplantation therapies is considered, the pluripotent stem cells according to the present invention could fall under the category of class II of regenerative medicine (middle risk), unlike iPS cells under the category of class I of regenerative medicine (high risk) under the Act on the Safety of Regenerative Medicine in Japan.

Furthermore, the method for producing pluripotent stem cells of the present invention can produce pluripotent stem cells in a shorter period of time than a method for producing pluripotent stem cells by direct reprogramming (about 60 days). Additionally, since the method for producing pluripotent stem cells of the present invention is direct reprogramming by forming spheroids, the method of the present invention has a wider range of application, for example, in selection of material tissues for establishment or expansion culture methods, than direct reprogramming by using compound cocktails.

The use of the pluripotent stem cells obtained by the production method of the present invention is not limited. The pluripotent stem cells can be used in various tests and research and treatment of diseases.

### Method for Producing Cancer Stem Cells

Another embodiment of the present invention provides a method for producing cancer stem cells, including culturing cancer cells by using the cell culture supplement of the present invention. Culturing cancer cells by using the cell culture supplement of the present invention allows the cancer cells to form spheroids, enabling the simple and efficient production of cancer stem cells.

The method using the cell culture supplement of the present invention in the production of cancer stem cells includes, but is not limited to, for example, using the cell culture supplement of the present invention to treat the culture surface of a cell culture substrate and/or adding the cell culture supplement of the present invention to a culture medium. Preferably, cancer cells are cultured by using a cell culture substrate having the culture surface treated with the cell culture supplement of the present invention. The use of the cell culture supplement for surface treatment of the culture surface of a cell culture substrate and the use by adding the cell culture supplement to a culture medium are the same as described in section "Use in Surface Treatment Agent or Additive" above.

The cancer cells used in the production of cancer stem cells of the present invention can be any cancer cells derived from a mammal (humans, mice, rats, cows, pigs, monkeys, sheep, rabbits, and dogs) and can be selected from a wide range. Of these, humans, monkeys, and mice are preferred, and humans are particularly preferred. The type of cancer is also not limited and can be selected from a wide range of those, such as bowel cancer, breast cancer, ovarian cancer, cervical cancer, endometrial cancer, prostate cancer, stomach cancer, liver cancer, esophagus cancer, pancreas cancer, bladder cancer, bile duct cancer, laryngeal cancer, melanoma, lung cancer, chronic lymphocytic leukemia, chronic myeloid leukemia, thyroid cancer, multiple myeloma, and sarcoma. A wide range of cells, including cell line-established cells, patient-specimen-derived primary culture cells, patient-specimen-derived subculturable cells (PDCC), animal transplanted cancer tissue-derived cancer cells (PDOX), and experimental animal-derived cancer cells.

The medium for use in the production of pluripotent stem cells can be selected from a wide range of those listed in section "Spheroid-forming Agent" above. Of these, a DMEM/F12 medium is preferred.

Additionally, the method for producing cancer stem cells of the present invention does not require adding various adjuvants for differential proliferation, such as cell growth factors (e.g., PDGF, TGF-β, Activin, BMP, Noggin, LIF, R-Spondin, Wnt-3a, EGF, TPO, SCF, and Neuregulin), mesenchymal stem cell maintenance mediums, and retinoic acid. Using the cell culture supplement of the present invention enables the production of cancer stem cells from cancer cells without adding cell growth factors to the medium.

The method for producing cancer stem cells can be performed according to a commonly used method for forming spheroids, except for performing cell culture by using a cell culture substrate having the cell culture supplement of the present invention on its culture surface. Examples include a non-adhesive surface cell culture method, a hanging-drop cell culture method, use of a micromolding technique, and a rotary cell culture method, with a non-adhesive surface cell culture method being particularly preferred.

The culture conditions for producing cancer stem cells are not limited. For example, the culture temperature may be 20 to 40°C, and preferably 34 to 38°C. The culture can also be performed in a 3 to 10% CO₂ atmosphere, and preferably in a 4 to 6% CO₂ atmosphere. The culture period is, for example, about 10 to 40 days, and preferably about 15 to 30 days. Optionally, the medium may be replaced one or more times during the culture period. Optionally, the cell culture substrate may be replaced one or more times during the culture period.

The size of a spheroid in the production of cancer stem cells is typically about 10 to 500 µm, and preferably about 50 to 200 µm in diameter. It is preferred to separate spheroids that have reached a suitable size because it is difficult for nutrients to penetrate into the interior of overly large spheroids. The resulting spheroids can be disassembled and then cultured again with the cell culture supplement of the present invention to form spheroids that have functionality of cancer stem cells again.

The concentration of cancer cells in culture is not limited as long as cancer stem cells are efficiently produced. For example, the concentration of cancer cells in culture is about 1 × 10³ to about 1 × 10⁷ cells/well, and preferably about 3 × 10³ to about 5 × 10⁶ cells/well.

In the method for producing cancer stem cells of the present invention, the production of cancer stem cells can be confirmed by identifying the expression of cancer cell surface markers. Examples of cancer cell surface markers include CD133, CD44, CD24, ABCB5, EAS, CD34, CD38, CD90, CD117, and CXCR4.

The cancer stem cells obtained by the production method of the present invention can also confirm the expression of undifferentiated stem cell markers. Examples of undifferentiated stem cell markers include OCT4, SOX1, SOX2, SOX3, SOX18, NANOG, Klf2, Klf4, Essrb, Sall4, Tell, Tcl3, c-MYC, c-MYCn, LIN28, SSEA-1, SSEA-3, SSEA-4, TRA-1-60, TRA-1-81, AP, Fzd1-10, and TDGF-1.

The cancer stem cells obtained by the production method of the present invention may be a population of heterogeneous cancer stem cells containing a variety of cancer stem cells, and the population of heterogeneous cancer stem cells may further contain cancer cells.

The use of the cancer stem cells obtained by the production method of the present invention is not limited. The cancer stem cells can be used in various tests and research and treatment of diseases.

### Induction of Stem Cell Differentiation

The pluripotent stem cells obtained by the production method of the present invention can be induced to differentiate into cells of endodermal organs (e.g., liver, pancreas, intestinal tract, lung, thyroid, parathyroid, urinary tract, adipose tissue, and cartilage tissue), mesodermal organs (e.g., kidney, ureter, heart, blood, gonad, adrenal cortex, muscle, skeleton, dermis, connective tissue, and mesothelia), or ectodermal organs (e.g., brain, spinal cord, adrenal medulla, epidermis, hair, nail, skin glands, sensory organs, peripheral nerves, and lens) according to a known method. Additionally, organoids and tissues of these organs can be produced according to a known method.

Differentiation induction can be performed as appropriate using any method for inducing differentiation into any cells with reference to a differentiation induction method reported for pluripotent stem cells such as ES cells and iPS cells. For example, induction of differentiation of pluripotent stem cells into hematopoietic lineage cells can be performed according to the method disclosed in the following document: Niwa A, Heike T, Saito K, et al. A novel serum-free monolayer culture for orderly hematopoietic differentiation of human pluripotent cells via mesodermal progenitors. PLoS One 2011; Issue 7, Vol. 6.

Organoids and tissues obtained by the method of the present invention can be used in evaluating the efficacy and toxicity of a test substance, elucidating the mechanism of action of a test substance, or analyzing the mechanism of biological phenomena.

### Examples

The following describes the present invention in more detail with reference to Examples. However, the present invention is not limited to the Examples.

### Example 1: Spheroid Culture of Healthy Human Neonatal Fibroblasts and Expression of Undifferentiated Stem Cell Marker Gene

### Method

2 mL of an aqueous solution of the polypeptide represented by formula (1) (0.125%) (cell culture supplement) was added to a commercial polystyrene-made 6-well flat-bottom culture vessel (CellBIND, Corning) and allowed to stand at 4°C for 24 hours.

Thereafter, the excess cell culture supplement was removed, and the remaining solution was dried in a dryer at 37°C for 96 hours to complete coating treatment with the cell culture supplement. Healthy human neonatal fibroblasts (ATCC PCS-201-010, primary dermal fibroblasts, neonatal, referred to as "fibroblasts" below) were maintained and cultured in a serum-free medium (Promo Cell; fibroblast basal medium, basic-FGF 5 ng/mL and insulin 2.5 µg/mL). The fibroblasts floating in 2 mL of the medium were seeded in the culture vessel coated with the cell culture supplement at 2 × 10⁶ cells/well and cultured at 37°C under 5% CO₂ conditions. For a control, fibroblasts were seeded in a culture vessel of the same type with no coating applied with the cell culture supplement (2 × 10⁶ cells/well). Observation was performed with a microscope on day 2 and day 20 after the start of culture to capture images of spheroid formation (Fig. 5).

The analysis of expression of gene mRNA was performed by real-time PCR. The fibroblasts were spheroid-cultured according to the same method as above. The spheroids were collected in a 15-mL centrifuge tube on day 20 and centrifuged. The cells were washed with phosphate buffered saline (PBS(-)) and collected. Cells cultured in the culture vessel not coated with the cell culture supplement (control) (day 0) were washed with PBS(-) after removal of the medium, and the cells were detached from the culture vessel with 0.05% trypsin-EGTA and dissociated, followed by collecting the cells in a 15-mL centrifuge tube and washing with PBS(-) again. RNA was extracted from the collected spheroids and the control cells and subjected to analysis of the expression of undifferentiated stem cell marker genes by real-time PCR.

Specifically, a NucleoZOL reagent (Macherey-Nagel) was added to each type of cells, and total-RNA was extracted according to the protocol recommended by the reagent manufacturer and used as template RNA for detecting undifferentiated stem cell markers. For real-time PCR analysis, PCR probes from QuantiFast Probe RT-PCR Kits (QIAGEN; No. 204454) and TaqMan Gene Expression Assay Probe (Thermo Fisher Scientific) were used. PCR analysis was performed with a Rotor-Gene Q (QIAGEN) apparatus. In addition to cytokines and chemokines expected to be involved in the formation of spheroids, the genes targeted for detection were the following: the three genes, SOX2 (sex-determining region Y-box 2), OCT4 (octamer binding transcription factor 4), and NANOG (homeodomain transcription factor), which are transcription factors used in the production of iPS cells as probes for detecting the expression of undifferentiated stem cell marker genes (NPL 1); β-actin was used as an endogenous control gene. In the gene expression analysis, relative quantification was performed according to the ΔΔCt method (Delta Delta Ct method). The results of analysis according to the ΔΔCt method show the amount of a gene expressed by control cells under the conditions of "no cell culture supplement, flat-bottom 2D growth, day 0" as "1.0" in relative terms, and the increase rate of the expression level on day 20 of spheroid proliferation was determined by comparison (Fig. 6).

### Results

Fig. 5 shows the results of spheroid culture of fibroblasts in the culture vessel treated with the cell culture supplement. In the control vessel, to which no cell culture supplement was applied, fibroblasts adhered to the bottom and grew two-dimensionally (day 2 in culture). In contrast, in vessels treated with cell culture supplement, the cells aggregated, formed spheroids and proliferated in three dimensions. Over a period of day 2 to day 20 from the start of culture, the cells forming spheroids proliferated, and the size of the spheroids also increased.

Fig. 6 shows the results of real-time PCR of gene mRNA. In the spheroids of fibroblasts on day 20 from the start of culture, the gene expression levels of marker proteins SOX2, OCT4, and NANOG, which define the characteristics of stem cells, were found to have increased, indicating that the cells acquired the characteristics of stem cells. Specifically, the gene expression levels of SOX2, OCT4, and NANOG in the fibroblasts cultured without the cell culture supplement (control) were defined as 1.0 in relative terms and compared with the gene expression levels of SOX2, OCT4, and NANOG in the three-dimensional spheroid culture on day 20 according to the ΔΔCt method. The results indicate that SOX2 increased 34.4-fold, OCT4 increased 5.1-fold, and NANOG increased 9.1-fold.

### Discussion

Fig. 5 indicates that if fibroblasts are cultured in a polypropylene-made cell culture vessel, the cells adhere to the bottom of the culture vessel and proliferate (2D adhesion proliferation) because the bottom of such a vessel is generally highly adhesive to cells due to hydrophilic or hydrophobic treatment. However, the cells did not adhere to the bottom surface of the culture vessel coated with an aqueous solution of the polypeptide represented by formula (1), but moved around and gathered to form spheroids, which are three-dimensional cell aggregates. Since the human body is constructed as a three-dimensional aggregate of cells in which biological phenomena take place, three-dimensional culture reflects tissue in vivo better than two-dimensional cultured cells, with various physiological functions and gene expressions controlled.

As shown in the results in Fig. 6, spheroid culture of fibroblasts in a culture vessel treated with the cell culture supplement was found to be able to induce stem cells with the characteristics of pluripotent stem cells without introducing genes used in the conventional iPS cell production method and without using compound cocktails used in direct-reprogramming with compounds.

In other words, the inventor found a novel spheroid culture method using a cell culture supplement, capable of inducing cells equipped with the characteristics of pluripotent stem cells from fibroblasts (somatic cells) by a novel direct-reprogramming mechanism.

The increase rate in the expression levels of mRNA of undifferentiated stem cell markers is not limited to these values. In addition to SOX2, OCT3/4, and NANOG, human undifferentiated stem cell markers such as Klf4, Essrb, Sall4, Lin28, SSEA, TRA-1-60, and c-MYC are also known. Thus, the present invention is not limited by these three genes described in the Examples.

### Example 2: Induction of Differentiation of Stem Cells Produced from Healthy Human Neonatal Fibroblasts into Primordial Three Germ Layers

### Method

To observe the spheroid formation and stem cell induction in healthy human neonatal fibroblasts over time, culture was performed by using a 96-well round-bottom culture vessel. An aqueous solution of the polypeptide represented by formula (1) (0.125%) (cell culture supplement) was added to a commercial polystyrene-made 96-well round-bottom culture vessel (Nunclon Delta 96U Bottom, Thermo Fisher Scientific) at 100 µL/well and allowed to stand at 4°C for 24 hours. Thereafter, the excess cell culture supplement was removed, and the remaining solution was dried in a dryer at 37°C for 96 hours to complete coating treatment with the cell culture supplement. Fibroblasts were maintained and cultured in a serum-free medium (Promo Cell; fibroblast basal medium, basic-FGF 5 ng/mL and insulin 2.5 µg/mL). The fibroblasts floating in the medium were seeded in the culture vessel coated with the cell culture supplement at 4 × 10⁴ cells/well/100 µL and cultured at 37°C under 5% CO₂ conditions.

The spheroid formation was observed with a microscope on day 0, 2, and 20 after the start of culture (Fig. 7).

To investigate the expression mechanism of the undifferentiated stem cell marker genes found in Example 1 over time, real-time PCR analysis was performed by using the following three transcription factors: SOX2 (sex-determining region Y-box 2), OCT4 (octamer binding transcription factor 4), and NANOG (homeodomain transcription factor), and a TaqMan gene expression assay probe (β-actin) as an endogenous control gene. In the expression analysis, relative quantification was performed according to the ΔΔCt method (Delta Delta Ct method)(Fig. 8).

To determine whether stem cells induced from fibroblasts have a differentiation-inducing ability, analysis was conducted on the expression of genes associated with the three germ layers that indicate the early stages of differentiation. Fibroblasts were cultured in the same type of a 96-well culture vessel treated with the cell culture supplement as above according to the same method. Real-time PCR analysis was performed by using the following expression detection probes for the genes associated with differentiation into the three germ layers: SOX17 (sex-determining region Y-box 17; endoderm), HAND1(Heart-and neural crest derivatives-expressed protein-1; mesoderm), and MAP2 (microtubule associated protein 2: ectoderm), and a TaqMan gene expression assay probe (β-actin) as the endogenous control gene. In the expression analysis, relative quantification was performed according to the ΔΔCt method (Delta Delta Ct method) (Fig. 9).

### Results

The results shown in Fig. 7 indicate that spheroids of fibroblasts in the 96-well round-bottom culture vessel grew over time. Due to the use of a 96-well round-bottom culture vessel treated with the cell culture supplement, the cells formed a single spheroid, allowing homogeneous measurement of gene expression and bioactivity for each spheroid.

Fig. 8 shows the results of real-time PCR of mRNA of undifferentiated stem cell markers. The results indicate that the expression levels of the undifferentiated stem cell markers SOX2, OCT4, and NANOG increased over time after day 5 from the start of culture of fibroblast spheroids.

Specifically, the gene expression levels of SOX2, OCT4, and NANOG in two-dimensionally adhesion-cultured fibroblasts without the cell culture supplement (control) (day 0) were defined as 1.0 in relative terms and compared with the gene expression levels of SOX2, OCT4, and NANOG on day 5, day 10, and day 20 of three-dimensional spheroid culture by ΔΔCt relative quantification. The expression levels of all of the genes increased over time and reached their highest levels on day 20, with SOX2 increasing 144.0-fold, OCT4 increasing 30.1-fold, and NANOG increasing 35.4-fold.

Fig. 9 shows the results of real-time PCR of mRNA of genes associated with the three germ layers. The results indicate that the expression levels of the genes of marker proteins SOX17, HAND1, and MAP2, which define the differentiation into three germ layers, increased after day 5 from the start of spheroid culture of fibroblasts.

Specifically, the gene expression levels of SOX17, HAND1, and MAP2 in two-dimensionally adhesion-cultured fibroblasts without the cell culture supplement (control) (day 0) were defined as 1.0 in relative terms and compared with the gene expression levels of SOX17, HAND1 and MAP2 on day 5, day 10, and day 20 of three-dimensional spheroid culture by ΔΔCt relative quantification. The expression levels of all of the genes increased over time and reached their highest levels on day 20, with SOX17 increasing 359.6-fold, HAND1 increasing 4.6-fold, and MAP2 increasing 24.2-fold.

### Discussion

Fig. 7 shows that stem cells with the characteristics of pluripotent stem cells can be produced from fibroblasts, which are somatic cells (direct-reprogramming). Furthermore, in regards to the shape of the culture vessel, the same results were shown to be achieved in a flat-bottom culture vessel and a round-bottom culture vessel.

Fig. 8 indicates that the use of a 96-well culture vessel treated with the cell culture supplement in spheroid culture of fibroblasts allows the cells to form a single homogeneous spheroid, enabling precise gene expression analysis. The results indicate that fibroblasts can be initialized (directly reprogrammed) into pluripotent stem cells with enhanced expression of SOX2, OCT4, and NANOG genes without the method of introducing transcription factor genes used in the production of iPS cells and without adding compound cocktails to a culture medium as used in direct-reprogramming with compounds. In other words, the inventor is considered to have invented a novel direct-reprogramming method that produces cells with the characteristics of pluripotent stem cells from fibroblasts (somatic cells) by using a spheroid culture method with the cell culture supplement.

The results shown in Fig. 9 indicate that stem cells produced from fibroblasts can be induced to differentiate into cells with the traits of an embryoid body, which is a three-dimensional cell clump. In general, induction of differentiation of a stem cell into a mature organ cell requires a process of inducing the stem cell to differentiate into a germ layer of the target organ lineage. The present culture method is considered to be a novel culture method that causes initialization (direct-reprogramming) of somatic cells into stem cells by a mechanism different from that of the known iPS cell method or direct-reprogramming method with chemical compounds, and that simultaneously achieves the induction of differentiation into embryoid bodies that are SOX17 (endoderm marker gene) positive, HAND1 (mesoderm marker gene) positive, and MAP2 (ectoderm marker gene) positive.

In addition to SOX2, OCT4, and NANOG, undifferentiated stem cell markers such as Klf4, Essrb, Sall4, Lin28, SSEA, and TRA-1-60 are also known. Thus, the present invention is not limited by these three genes. Similarly, because other triploblastic marker genes are known in addition to SOX17, HAND1, and MAP2, the present invention is not limited by these marker genes. Furthermore, the increase in the gene expression levels of the undifferentiated stem cell markers as well as the triploblastic markers is not limited to these values.

### Example 3: Expression of Stem Cell Marker Protein in Healthy Human Neonatal Fibroblast Spheroid

### Method

To confirm whether stem cells were induced in fibroblast spheroids, the expression of stem cell marker proteins was examined by immunostaining with fluorescent dye-labeled antibodies. An aqueous solution of the polypeptide represented by formula (1) (0.125%) (cell culture supplement) was added to a commercial polystyrene-made 96-well round-bottom culture vessel (Nunclon Delta 96U Bottom, Thermo Fisher Scientific) at 100 µL/well, and the coating treatment with the cell culture supplement was completed in the same manner as in Example 2. Fibroblasts were maintained and cultured in a serum-free medium (Fujifilm Wako Pure Chemical Corporation; DMEM/F12 medium, human serum albumin recombinant 0.5 mg/mL, human transferrin recombinant 10 ug/mL, basic-FGF 5 ng/mL, and insulin 2.5 µg/mL). Fibroblasts floating in the medium were seeded in the culture vessel coated with the cell culture supplement at 4 × 10⁴ cells/well/100 µL and cultured at 37°C under 5% CO₂ conditions. Spheroids were collected from the 96-well round-bottom culture vessel on day 20 from the start of culture, and cell fixation was performed with a 4% paraformaldehyde solution, followed by immunostaining. For fluorescence-labeled antibodies, three antibodies for detection of human stem cell marker proteins (NL557 fluorescent dye-labeled anti-SOX2 antibody (fluorescent orange), NL493 fluorescent dye-labeled anti-NANOG antibody (fluorescent green), and NL637-labeled anti-OCT4 antibody (fluorescent red), R&D Systems)) were used and reacted simultaneously to perform observation with a fluorescence microscope.

### Results

Fig. 10 shows fluorescence microscope photographs of immunostained fibroblast spheroids. Fluorescence indicating the stem cell marker proteins SOX2 (orange), OCT4 (red), and NANOG (green) was detected, indicating that stem cells expressing the three stem cell marker proteins were induced in the cells forming spheroids.

### Discussion

In Figs. 6 and 8, real-time PCR analysis indicates that the cells forming spheroids express undifferentiated stem cell marker genes. Marker proteins produced from undifferentiated marker genes were also found to have been expressed in fibroblast spheroids. The presence of stem cells was confirmed in aspects of gene and protein.

### Example 4: Detection of Stem Cells in Healthy Human Neonatal Fibroblast Spheroid

### Method

To detect stem cells induced in fibroblast spheroids, immunostaining with a fluorescent dye-labeled antibody was used, and stem cells in the spheroid-forming cells were detected. An aqueous solution of the polypeptide represented by formula (1) (0.125%) (cell culture supplement) was added to a commercial polystyrene-made 96-well round-bottom culture vessel (Nunclon Delta 96U Bottom, Thermo Fisher Scientific) at 100 µL/well to complete coating treatment with the cell culture supplement in the same manner as in Example 2. Fibroblasts were maintained and cultured in a serum-free medium (Fujifilm Wako Pure Chemical Corporation, DMEM/F12 medium, human serum albumin recombinant 0.5 mg/mL, human transferrin recombinant 10 µg/mL, basic-FGF 5 ng/mL, and insulin 2.5 µg/mL). Fibroblasts floating in the medium were seeded in the culture vessel coated with the cell culture supplement at 2 × 10⁴ cells/well/100 µL and cultured at 37°C under 5% CO₂ conditions. On day 20 after the start of culture, spheroids were aseptically collected from the 96-well round-bottom culture vessel. The cells were dissociated with an Accutase enzyme reagent and transferred to an incubator for immunostaining (Millicell Chamber, Millipore) to continue culture in the same medium. After 24 hours, the cells were fixed with a 4% paraformaldehyde solution and immunostained. For the fluorescence-labeled antibody, NL493 fluorescent dye-labeled anti-NANOG antibody (fluorescent green, R&D systems) was used, and observation was performed with a fluorescence microscope.

### Results

Fig. 11 shows the presence of NANOG protein-positive stem cells in a cell population obtained by dispersing spheroids. In Fig. 11, the majority of fibroblast nuclei was NANOG-negative, but the nuclei of some cells showed strong NANOG antibody-positive green fluorescence, indicating that stem cell marker cells were induced.

### Discussion

The NANOG protein is a homeobox transcription factor, which is made in the cytoplasm and translocated into the nucleus, and plays an important role in maintaining stem cell pluripotency. In addition to the identification of stem cell marker proteins in all of the spheroids (Fig. 10), the study also showed that stem cells positive for nuclear NANOG protein can be induced at the cellular level.

### Example 5: Production of Stem Cells by Spheroid Culture of Human Adult Skin Fibroblast

### Method

To induce stem cells from healthy human adult skin fibroblasts (direct-reprogramming), adult skin fibroblasts were cultured to form spheroids in the same manner as in Example 2. An aqueous solution of the polypeptide represented by formula (1) (0.125%) (cell culture supplement) was added to a commercial polystyrene-made 96-well round-bottom culture vessel (Nunclon Delta 96U Bottom, Thermo Fisher Scientific) and allowed to stand at 4°C for 24 hours. The cell culture supplement solution was then removed and the coated cell culture supplement was dried in a dryer at 37°C for 96 hours to complete the cell culture supplement coating process. Healthy human adult skin fibroblasts (Lonza, normal human dermal fibroblast-adult, referred to as "adult skin fibroblasts" below) were maintained and cultured in a serum-free medium (Fujifilm Wako Pure Chemical Corporation; DMEM/F12 medium, human serum albumin recombinant 0.5 mg/mL, human transferrin recombinant 10 ug/mL, basic-FGF 5 ng/mL, and insulin 2.5 µg/mL). Adult skin fibroblasts floating in 100 µL of the medium were seeded in the 96-well round-bottom culture vessel coated with the cell culture supplement at 2 × 10⁴ cells/well and cultured at 37°C under 5% CO₂ conditions.

The detection of stem cell marker genes was performed by analysis of the expression of mRNA by real-time PCR. Specifically, on day 20 from the start of culture, spheroids were collected in a 15-mL centrifuge tube and centrifuged, followed by washing the cells with phosphate buffered saline(-) and collecting the cells. Cells two-dimensionally cultured in a culture vessel not treated with the cell culture supplement (control) (day 0) were washed with PBS(-) after removal of the medium. The cells were detached from the plate with 0.05% trypsin-EGTA and dissociated, followed by collecting the cells in a 15-mL centrifuge tube and washing again with PBS(-) again. A NucleoZOL (Macherey-Nagel) reagent was added to the spheroids, and RNA was extracted according to the protocol recommended by the reagent manufacturer and determined to be template RNA. For real-time PCR analysis, PCR probes from QuantiFast Probe RT-PCR Kits (QIAGEN; No. 204454) and TaqMan Gene Expression Assay Probe (Thermo Fisher Scientific) were used. PCR analysis was performed with a Rotor-Gene Q (QIAGEN) apparatus. For detection of genes, three transcription factors were used as probes for detecting the expression of undifferentiated stem cell marker genes: SOX2 (sex-determining region Y-box 2), OCT4 (octamer binding transcription factor 4), and NANOG (homeodomain transcription factor); and β-actin was used as an endogenous control gene (Fig. 13).

To determine whether stem cells induced in spheroids can differentiate into primordial three germ layers, the expression of triploblastic marker genes was examined by real-time PCR. For probes for detection, the following four triploblastic marker genes were used: SOX17 (sex-determining region Y-box 17; endoderm), GATA6 (GATA family of zinc finger transcription factors; endoderm), HAND1 (heart- and neural crest derivatives-expressed protein 1; mesoderm), and SOX1 (HMG-box (high mobility group) DNA-binding domain; ectoderm). For an endogenous control gene, TaqMan Gene Expression Assay Probe (β-actin) was used (Fig. 14). In the gene expression analysis, relative quantification was performed according to the ΔΔCt method (Delta Delta Ct method). The results of analysis according to the ΔΔCt method show the amount of a gene expressed by control cells under the conditions of "no cell culture supplement, flat-bottom 2D growth, day 0" as "1.0" in relative terms, and the increase rate in "the expression level in spheroid proliferation on day 20" was determined by comparison.

### Results

Fig. 12 shows the results of spheroid culture of adult skin fibroblasts with the culture vessel treated with the cell culture supplement. In the control vessel, to which no cell culture supplement was applied, adult skin fibroblasts adhered to the bottom and grew two-dimensionally (day 2 in culture). However, in the culture vessel coated with the cell culture supplement, the cells formed spheroids and proliferated three-dimensionally.

Fig. 13 shows the results of real-time PCR of gene mRNA. In the adult skin fibroblast spheroids on day 20 from the start of culture, the gene expression levels of marker proteins SOX2, OCT4, and NANOG, which define the characteristics of stem cells, were found to have increased, indicating that the cells acquired the characteristics of stem cells. Specifically, the gene expression levels of SOX2, OCT4, and NANOG in two-dimensionally cultured control cells were defined as 1.0 in relative terms and compared with the gene expression levels of SOX2, OCT4, and NANOG of the three-dimensional spheroid culture on day 20 according to the ΔΔCt method. The results indicate that SOX2 increased 9.4-fold, OCT4 increased 13.0-fold, and NANOG increased 50.5-fold.

Similarly, Fig. 14 shows increases in the expression levels of marker proteins SOX17, GATA6, HAND1, and SOX1, which define the differentiation into three germ layers. The gene expression levels of SOX17, GATA6, HAND1, and SOX1 in the control cells were defined as 1.0 in relative terms and compared with the gene expression levels of SOX17, GATA6, and MAP2 of the three-dimensional spheroid culture on day 20 by ΔΔCt relative quantification. The expression levels of all genes increased, with SOX17 increasing 11.4-fold, GATA6 increasing 2.2-fold, HAND1 increasing 33.9-fold, and SOX1 increasing 7.1-fold.

### Discussion

For the somatic-cell direct reprogramming technology using compounds, there have been reports on experiments to induce stem cells from fetal fibroblasts or adult fibroblasts in mice. However, an applied technology for fibroblasts derived from human adults has not yet been established. This study is considered to have found a stable and reproducible method of producing stem cells from adult skin fibroblasts as well as from human neonatal fibroblasts by using the cell culture supplement of the present invention.

The spheroid culture method using the cell culture supplement can induce stem cells from skin-derived fibroblasts, which are easy to collect with minimum invasion from individual patients. Thus, this will be a practical technology for individualized regenerative therapies and screening for personalized diagnostic methods and therapeutic drugs.

### Example 6: Spheroid Culture of Healthy Human Adult Mammary Gland Epithelial Cells and Expression of Undifferentiated Stem Cell Marker Gene

### Method

An aqueous solution of the polypeptide represented by formula (1) (0.125%) (cell culture supplement) was added to a commercial polystyrene-made 6-well flat-bottom culture vessel (CellBIND, Corning) and to a polystyrene-made 96-well round-bottom culture vessel (Nunclon Delta 96U Bottom, Thermo Fisher Scientific) and allowed to stand at 4°C for 24 hours.

The cell culture supplement solution was then removed and the coated cell culture supplement was dried in a dryer at 37°C for 96 hours to complete the cell culture supplement coating process. Healthy human adult mammary gland epithelial cells (ATCC PCS-600-010, primary mammary epithelial cells, referred to as "mammary gland epithelial cells" below) were maintained and cultured in a serum-free medium (Promo Cell; mammary epithelial cell growth medium, EGF 5 ng/ mL, and insulin 2.5 µg/mL). In the culture vessels coated with the cell culture supplement, the mammary gland epithelial cells floating in 2 mL of the medium were seeded at 2 × 10⁶ cells/well (6-well flat-bottom culture vessel) and the mammary gland epithelial cells floating in 100 µL of the medium were seeded at 2 × 10⁴ cells/well (96-well round-bottom culture vessel). The cells of each culture vessel were cultured at 37°C under 5% CO₂ conditions. For a control, mammary gland epithelial cells were seeded in the same type of culture vessel with no coating with the cell culture supplement under the same conditions. Observation was performed with a microscope on day 2 and day 20 after the start of culture to capture images of spheroid formation (Fig. 15).

To determine whether stem cells could be induced (direct-reprogrammed) from mammary gland epithelial cells (somatic cells), mammary gland epithelial cells were cultured in a 96-well culture vessel treated with the cell culture supplement to form spheroids. The analysis of expression of gene mRNA was performed by real-time PCR.

Specifically, on day 20 from the start of culture, spheroids were collected in a 15-mL centrifuge tube and centrifuged, and then washed with phosphate buffered saline (-) and collected. Two-dimensionally cultured cells in the culture vessel that was not coated with the cell culture supplement (control) (day 0) were washed with PBS(-) after removal of the medium, and the cells were detached from the plate with 0.05% trypsin-EGTA and dissociated, followed by collecting the cells in a 15-mL centrifuge tube and washing with PBS(-) again. A NucleoZOL reagent (Macherey-Nagel) was added to the spheroids, and RNA extracted according to the protocol recommended by the reagent manufacturer was determined to be template RNA. For real-time PCR analysis, PCR probes from QuantiFast Probe RT-PCR Kits (QIAGEN; No. 204454) and TaqMan Gene Expression Assay Probe (Thermo Fisher Scientific) were used. PCR analysis was performed with a Rotor-Gene Q (QIAGEN) apparatus. The genes targeted for detection were the following three transcription factors: SOX2 (sex-determining region Y-box 2), OCT4 (octamer binding transcription factor 4), and NANOG (homeodomain transcription factor) as the probes for detecting the expression of undifferentiated stem cell marker genes; and β-actin was used as an endogenous control gene (Fig. 16).

For probes for detection of genes associated with primordial three germ layers, the following three triploblastic marker genes were used: SOX17 (sex-determining region Y-box 17; endoderm), GATA6 (GATA family of zinc finger transcription factors; mesoderm), and MAP2 (microtubule associated protein 2: ectoderm). For an endogenous control gene, TaqMan Gene Expression Assay Probe of β-actin was used (Fig. 17).

In the gene expression analysis, relative quantification was performed according to the ΔΔCt method (Delta Delta Ct method). The results of analysis according to the ΔΔCt method show the amount of a gene expressed by control cells under the conditions of "no cell culture supplement, flat-bottom 2D growth, day 0" as "1.0" in relative terms, and the increase rate in "the expression level in spheroid proliferation on day 20" was determined by comparison.

### Results

Fig. 15 shows the results of spheroid culture of mammary gland epithelial cells in the culture vessel treated with the cell culture supplement. In the control vessel, to which no cell culture supplement was applied, mammary gland epithelial cells adhered to the bottom and showed two-dimensional planar growth (day 2 in culture). However, in the culture vessel coated with the cell culture supplement, the cells self-assembled to form spheroids and proliferated three-dimensionally. Over a period of day 2 to day 20 from the start of culture, the cells forming spheroids proliferated with increase in size of spheroids.

Fig. 16 shows the results of real-time PCR analysis of mRNA to examine the expression of undifferentiated stem cell marker genes induced (direct-reprogrammed) from mammary gland epithelial cells. The results indicated that the gene expression levels of undifferentiated stem cell marker proteins SOX2, OCT4, and NANOG increased on day 20 from the start of spheroid culture as compared with those in two-dimensional adhesion culture of mammary gland epithelial cells (day 0).

Specifically, the gene expression levels of SOX2, OCT4, and NANOG in mammary gland epithelial cells in a two-dimensional adhesion culture without the cell culture supplement (control) were defined as 1.0 in relative terms and compared with the gene expression levels of SOX2, OCT4, and NANOG of the three-dimensional spheroid culture on day 20 by ΔΔCt relative quantification. The expression levels of all of the genes were found to have increased on day 20, with
SOX2 increasing 24.0-fold, OCT4 increasing 1.5-fold, and NANOG increasing 28.7-fold.

Fig. 17 shows the results of real-time PCR of gene mRNA associated with the primordial three germ layers. On day 20 from the start of spheroid culture of mammary gland epithelial cells, the gene expression levels of SOX17 and MAP2, among the marker genes that define differentiation into three germ layers, were found to have increased.

Specifically, the gene expression levels of SOX17, GATA6, and MAP2 in two-dimensionally adhesion-cultured mammary gland epithelial cells without the cell culture supplement (control) (day 0) were defined as 1.0 in relative terms and compared with the gene expression levels of SOX17, GATA4, and MAP2 of three-dimensional spheroid culture on day 20 by ΔΔCt relative quantification. On day 20 from the start of culture, SOX17 was found to have increased 124.7-fold, and MAP2 was found to have increased 2.7-fold. However, no gene expression of GATA6 (mesoderm marker) was detected.

### Discussion

The results shown in Fig. 15 indicate that stem cells with the characteristics of pluripotent stem cells can also be produced (direct-reprogrammed) from adult mammary gland epithelial cells. Furthermore, in regards to the shape of the culture vessel, the same results were shown to be achieved in a flat-bottom culture vessel and in a round-bottom culture vessel.

Fig. 16 indicates that the use of a 96-well culture vessel treated with the cell culture supplement in spheroid culture of mammary gland epithelial cells allows mammary gland epithelial cells to form a single homogeneous spheroid, enabling precise gene expression analysis. The results indicate that mammary gland epithelial cells can be initialized (directly reprogrammed) into pluripotent stem cells with enhanced expression of SOX2, OCT4, and NANOG genes without the method of introducing transcription factor genes used in the production of iPS cells and without adding compound cocktails to a culture medium as used in direct-reprogramming with compounds. In other words, the inventor is considered to have invented a novel direct-reprogramming method for producing cells with the characteristics of pluripotent stem cells from mammary gland epithelial cells (somatic cells) by using a spheroid culture method with the cell culture supplement.

The results shown in Fig. 17 indicate that the stem cells produced from mammary gland epithelial cells according to the present method can be induced to differentiate into three germ layers, which are in the early stage of differentiation into different organs. In general, induction of differentiation of a stem cell into a mature organ cell requires a process of inducing the stem cell to differentiate into a germ layer of the target organ lineage. The present culture method is considered to be a novel culture method that causes initialization (direct-reprogramming) of somatic cells into stem cells by a mechanism different from that of the known iPS cell method or direct-reprogramming method with compounds, and that simultaneously achieves the induction of differentiation into embryoid bodies that are SOX17 (endoderm marker gene) positive and MAP2 (ectoderm marker gene) positive.

In addition to SOX2, OCT4, and NANOG, undifferentiated stem cell marker genes such as Klf4, Essrb, Sall4, and Lin28 are also known. Thus, the present invention is not limited by these three genes. Similarly, because other primordial triploblastic marker genes are known in addition to SOX17, GATA4, and MAP2, the present invention is not limited by these marker genes. Furthermore, the increase in the gene expression levels of the stem cell markers as well as the primordial triploblastic markers is not limited to these values.

### Example 7: Two-dimensional Adhesion Culture and Three-dimensional Spheroid Culture of Cancer Cells

### Method

2 mL of an aqueous solution of the polypeptide represented by formula (1) (0.125%) (cell culture supplement) was added to a commercial polystyrene-made 6-well flat-bottom culture vessel (CellBIND, Corning) and allowed to stand at 4°C for 24 hours.

The cell culture supplement solution was then removed and the coated cell culture supplement was dried in a dryer at 37°C for 96 hours to complete the cell culture supplement coating process. For human cancer cells, common cancer cell lines, A549 cells (human lung cancer cells, ATCC CCL-185, carcinoma), ES2 cells (human ovarian cancer cells, ATCC CRL-1978, clear cell carcinoma), and HOS-143 cells (human osteosarcoma cells, ATCC CRL-8303, osteosarcoma), were used. Each type of cancer cells was seeded in the culture vessel at 2 × 10⁵ cells/well/100 µL and cultured at 37°C under 5% CO₂. The medium for use was a DMEM medium supplemented with 10% FBS and penicillin/streptomycin. For a control, cancer cells were seeded in the same type of culture vessel that was not coated with the cell culture supplement at 2 × 10⁶ cells/well. Observation was performed with a microscope on day 4 and day 15 after the start of culture to capture images of spheroid formation (Fig. 18).

### Results

Fig. 18 shows the results. In the culture vessel that was not coated with the cell culture supplement, cells adhered to the bottom and grew (two-dimensional adhesion proliferation). However, in the culture vessel coated with the cell culture supplement, the cells formed aggregates and then formed a few spheroids. Over a period of day 4 to day 15 from the start of culture, the cells forming spheroids proliferated, and the size of the spheroids also increased.

### Discussion

As seen in the results of Examples 1 to 6, spheroids were clearly shown to have formed in cancer cells, as well. Compared to two-dimensionally cultured cancer cells, three-dimensionally spheroid-cultured cancer cells are thought to exhibit characteristics similar to those of pathologic conditions in vivo. In other words, if cancer cells can be cultured three-dimensionally, a model that reflects the pathologic conditions of cancer can be created in a culture vessel.

Because the cell culture supplement containing the polypeptide represented by formula (1) was able to allow three cancer cell lines, A549 cells (lung cancer), ES2 (ovarian cancer), and HOS-143B (osteosarcoma), to form spheroids in the same manner, the reaction is not considered to be limited to these three cell types and is considered to be a method capable of providing a wide range of cancer models in vitro (in a culture vessel).

### Example 8: Study of Concentration of Cell Culture Supplement

### Method

An aqueous solution of the polypeptide represented by formula (1) (0.5%) was diluted 4-fold to 1024-fold to prepare different diluted solutions (cell culture supplement), and 100 ul of the solution was added to each well of a commercial 96-well flat-bottom plate (CellBIND, Corning) and allowed to stand at 4°C for 16 hours.

Thereafter, the excess cell culture supplement was removed, and the remaining solution was dried under reduced pressure at room temperature for 24 hours. HOS-143B (human osteosarcoma) was seeded at 3,000 cells/well in a culture plate coated with the cell culture supplement and cultured at 37°C under 5% CO₂ conditions. The medium used was DMEM + 10% FBS.

After 3 days of culture, the cells were observed with a microscope.

### Results

Fig. 19 shows the results. The formation of spheroids was observed in coated plates containing up to a 256-fold diluted solution of the aqueous solution of the polypeptide represented by formula (1). No formation of spheroids was observed in further diluted solutions.

### Discussion

The spheroid-forming capability of the aqueous solution of the polypeptide represented by formula (1) was found to be concentration-dependent.

### Example 9: Study on Plate Shape

### Method

200 µl of an aqueous solution of the polypeptide represented by formula (1) (0.125%) (cell culture supplement) was added to each well of a commercial 96-well flat-bottom plate (Thermo Fisher Scientific), a V-bottom plate (Thermo Fisher Scientific), and a U-bottom plate (Thermo Fisher Scientific) and allowed to stand at 4°C for 16 hours.

Thereafter, the excess cell culture supplement was removed, and the remaining solution was dried under reduced pressure at room temperature for 24 hours.

HeLa cells (human cervical epithelial cancer, JCRB9004, National Institutes of Biomedical Innovation, Health and Nutrition) and HOS-143B (human osteosarcoma) were seeded at 2,000 cells/well in a culture plate coated with the cell culture supplement and cultured at 37°C under 5% CO₂ conditions. The medium used was DMEM + 10% FBS. After 2 days of culture, the cells were observed with a microscope.

### Results

Fig. 20 shows the results. Cell attachment and proliferation were observed in the uncoated wells. Cell attachment and proliferation were observed in the uncoated wells. However, a single spheroid was found to have formed in the V-bottom plate coated with an aqueous solution of the polypeptide represented by formula (1), and several spheroids were found to have formed in the U-bottom plate. Numerous small spheroids were found to have formed in the flat-bottom plate.

### Discussion

The plates coated with the polypeptide represented by formula (1) were shown to form spheroids (cell clumps) regardless of plate shape.

### Example 10: Study on Inhibition of Induction of Apoptosis

### Method

An aqueous solution of the polypeptide represented by formula (1) was added in two different concentrations (prepared by diluting a 0.5% solution 4-fold and 8-fold) at 100 µL/well and 200 µL/well respectively to two 96-well cell culture plates with different bottom shapes (plate #163320 with a U-shaped bottom; Corning, NY, USA, and plate #277143 with a V-shaped bottom; Thermo Fisher Scientific, MA, USA) and allowed to stand overnight at 4°C. Thereafter, the excess cell culture supplement was removed, and the remaining solution was dried in a clean bench for about 6 hours to complete the treatment with the cell culture supplement. Two types of cell lines (canine tubule-derived MDCK II cells and HOS-143B cells) were seeded at 3,000 cells/well/100 µL for each and cultured in an incubator at 37°C in a 5% CO₂ atmosphere for 6 days. The control was cultured in a plate not treated with the cell culture supplement. After three days and six days, the apoptosis activity per well was measured by using a Caspase activity assay kit (Apo-ONE Homogeneous Caspase-3/7 Assay kit; Promega, WI, USA). Specifically, a fluorescent precursor substrate, bis-(n-CBZ-L-aspartyl-L-glutamyl-L-valyl-aspartic acid amide)rhodamine 110 (z-DEVD-rhodamine 110), was diluted 100-fold with a buffer provided in the kit and added to the culture plates in an amount of 50 µL per well. The plates were then gently shaken so as not to collapse the spheroids in the wells to mix the diluted substrate, and allowed to stand for 1 hour at room temperature. Thereafter, 100 µL of the culture fluid was transferred to a black 96-well cell culture plate (#237105; Thermo Fisher Scientific, MA, USA), and the value of fluorescence at 590 nm (excitation light at 560 nm) was measured with a plate reader. The value determined by subtracting the value for reagent-only with no cells (blank value) from a measured value was considered to be the measured value for each day of culture, and the values compared to those of the plate not coated with the aqueous solution of the polypeptide represented by formula (1) (control) were plotted.

### Results

Figs. 21 and 22 show the results. Apoptosis was found to be inhibited in both MDCK II cells and HOS-143B cells, compared with the cells cultured in the plate not coated with the aqueous solution of the polypeptide represented by formula (1), depending on the concentration of the polypeptide represented by formula (1). The apoptosis inhibition effect was more pronounced on day 6 in culture.

### Discussion

Cell death due to anoikis in primary cultured cells, apoptosis in the central part of spheroids occurring in spheroid-forming cells, and decreases in live cells due to the induction of necrosis in three-dimensional spheroid culture have been major problems. However, the cell culture supplement of the present invention inhibits the induction of apoptosis during spheroid formation in cells and was thus found to be extremely useful not only for stem cell production using spheroids, but also for drug discovery research and medical applications.

### Example 11: Detection of Gene Expression of Cancer Stem Cell Marker

### Method

A culture vessel treated with the cell culture supplement was prepared in the same manner as in Example 7, and the same three types of cancer cells as those in Example 7 were cultured. On day 4 and day 15 after the start of culture, spheroids of each of the three types of cancer cells were taken from the culture vessel and collected in an individual 15-mL centrifuge tube, followed by washing with phosphate buffered saline(-). After removal of the medium, cancer cells cultured in the plate free of the cell culture supplement (control) were washed with PBS(-), detached from the plate with 0.05% trypsin-EGTA and dissociated. The cancer cells were then collected in a 15-mL centrifuge tube and washed again with PBS(-). mRNA was extracted from the collected spheroids and control cells, and the expression of cancer stem cell marker genes was analyzed by real-time PCR.

Specifically, a NucleoZOL reagent (Macherey-Nagel) reagent was added to each type of cells, and RNA was extracted according to the protocol recommended by the reagent manufacturer and used as template RNA for detecting stem cell markers. For real-time PCR analysis, PCR probes from QuantiFast Probe RT-PCR Kits (QIAGEN; No. 204454) and TaqMan Gene Expression Assay Probe (Thermo Fisher Scientific) were used. PCR analysis was performed with a Rotor-Gene Q (QIAGEN) apparatus. For probes for detecting the expression of cancer stem cell marker genes, the following three PCR probes were used: CD24 (mucin-type glycoprotein, ligand for P-selectin, Oncogene), CD44 (cell surface glycoprotein involved cell-cell interaction, adhesion and migration, cancer stem cell marker), CD133 (five transmembrane glycoprotein, stem cell marker and cancer stem cell marker protein). B-actin was used as an endogenous control gene. For probes for detecting the expression of undifferentiated stem cell marker genes, three transcription factors, SOX2 (sex-determining region Y-box 2), OCT4 (octamer binding transcription factor 4), and NANOG (homeodomain transcription factor), which are used in the production of iPS cells, were used; and β-actin was used as the endogenous control gene. In the gene expression analysis, relative quantification was performed according to the ΔΔCt method (Delta Delta Ct method). The results of analysis according to the ΔΔCt method show the amount of a gene expressed by control cells under the conditions of "no cell culture supplement, 2D adhesion proliferation, day 4" as "1.0" in relative terms, and the increase rate in "the expression level in spheroid proliferation on day 15" was determined by comparison (Figs. 23 to 25) .

### Results

Figs. 23, 24, and 25 show the results of expression of cancer stem cell marker genes.

In Fig. 23, the gene expression levels of cancer stem cell markers CD24, CD44, and CD133 in A549 lung cancer cells were found to increase with time during culture (from day 4 to day 15) .

Specifically, CD24 increased 2.2-fold on day 4 and 4.2-fold on day 15 in spheroid culture. CD44 increased 1.6-fold on day 4 and 2.9-fold on day 15. CD133 increased 3.4-fold on day 4 and 13.5-fold on day 15.

In Fig. 24, the gene expression levels of CD24 in ES2 ovarian cancer cells increased 11.6-fold on day 4 and 63.2-fold on day 15 in spheroid culture. CD44 increased 1.3-fold on day 4 and 2.9-fold on day 15. CD133 was not detected in adhesion proliferation cells; instead, as compared with CD133 on day 4 in spheroid culture taken as the control value (1.0), CD133 increased 8.5-fold on day 15.

In Fig. 25, CD24 in HOS-143B osteosarcoma cells increased 1.34-fold on day 4 and 1.2-fold on day 15. CD44 increased 2.3-fold on day 4 and 1.9-fold on day 15. CD133 decreased 0.3-fold on day 4 but increased 2.1-fold on day 15.

### Discussion

A549 cells, ES2 cells, and HOS-143 cells are cancer cell lines established as cells to adhesion-proliferate. In the hierarchy of cancer cell differentiation, these cells are classified as "differentiated cancer cell lines" that have progressed to a higher degree of differentiation from cancer stem cells. Thus, cells cultured in a normal two-dimensional adhesion culture are known to express no cancer stem cell marker genes of CD24, CD44, and CD133 or to express these genes at very low levels. This study found that when these cancer cells were cultured with the cell culture supplement of the present invention, the cancer cells formed three-dimensionally structured spheroids and proliferated, and exhibited increased expression levels of CD24, CD44, and CD133 genes, which are indicators of cancer stem cells, during day 4 to day 15 from the start of culture.

Traditionally, to obtain cancer stem cells, cancer tissue removed from a cancer patient specimen has been transplanted into mice, or a small amount of cancer stem cells fractionated from cancer tissue with a cell sorter has been grown in a culture fluid supplemented with many expensive growth factors and hormones over a long period of time. Under such circumstances, the culture method of the present invention is considered to be a simple method for producing a large amount of cancer stem cells by culturing cells with only a cell culture supplement.

In addition to CD24, CD44, and CD133, other cancer stem cell markers are also known, and the present invention is not limited by these Examples.

### Example 12: Detection of Gene Expression of Undifferentiated Stem Cell Marker Transcription Factor in Cancer Cells

### Method

Coating of a culture vessel was completed in the same manner as in Examples 7 and 11, and three types of cancer cells, A549 cells, ES2 cells, and 143B cells, were cultured. For probes for detecting the expression of undifferentiated stem cell marker genes, the following three transcription factors were used: SOX2 (sex-determining region Y-box 2), OCT4 (octamer binding transcription factor 4), and NANOG (homeodomain transcription factor); and β-actin was used as an endogenous control gene. In the gene expression analysis, relative quantification was performed according to the ΔΔCt method (Delta Delta Ct method). The results of analysis according to the ΔΔCt method show the amount of a gene expressed by control cells under the conditions of "no cell culture supplement, 2D adhesion proliferation, day 4" as "1.0" in relative terms, and the increase rate in "the expression level in spheroid proliferation on day 15" was determined by comparison (Figs. 26 to 28).

### Results

Fig. 26 shows that the gene expression levels of undifferentiated stem cell markers SOX2, OCT4, and NANOG in A549 lung cancer cells increased over time (from day 4 to day 15).

Specifically, SOX2 increased 5.0-fold on day 4 and 6.0-fold on day 15. OCT4 increased 1.6-fold on day 4 and 3.7-fold on day 15. NANOG increased 10.6-fold on day 4 and 55.7-fold on day 15.

In Fig. 27, SOX2 in ES2 ovarian cancer cells was unchanged (1.1-fold) on day 4 and increased 3.4-fold on day 15. OCT4 increased 2.6-fold on day 4 and 4.6-fold on day 15. NANOG increased 3.9-fold on day 4 and 10.9-fold on day 15.

In Fig. 28, SOX2 in HOS-143B osteosarcoma cells increased 3.5-fold on day 4 and 1.6-fold on day 15. OCT4 increased 2.5-fold on day 4 and 2.4-fold on day 15. NANOG increased 1.6-fold on day 4 and 1.4-fold on day 15.

### Discussion

Undifferentiated stem cell marker genes such as SOX2, OCT4, and NANOG are clinically known to be expressed in some cancer tissues. Since the transcription factors of SOX2, OCT4, and NANOG have been the undifferentiated stem cell markers of ES cells and iPS cells, cancer cells with expression of these genes are thought to have the characteristics of "cancer stem cells." Undifferentiated stem cell marker genes such as SOX2, OCT4, and NANOG are known to be not expressed or to be expressed at very low levels in A549, ES2, or HOS-143 cells used in this study. Under such circumstances, the study found that when these three types of cancer cells were cultured with the cell culture supplement of the present invention, the cancer cells formed three-dimensionally structured spheroids and proliferated, and exhibited increased expression levels of SOX2, OCT4, and NANOG genes, which are markers of stem cells, during day 4 to day 15 of culture.

As noted above, the inventor came to invent a method for producing cancer stem cells in which cancer cells are cultured with the cell culture supplement of the present invention to allow undifferentiated stem cell marker genes of SOX2, OCT4, and NANOG to be expressed simply in a large amount, with expression of cancer stem cell marker genes CD24, CD44, and CD133.

In addition to SOX2, OCT4, and NANOG, other undifferentiated cancer stem cell markers are also known. The present invention is not limited by these Examples.

## Claims

1. A cell culture supplement comprising a polypeptide represented by the following formula (1):
(Pro-Hyp-Gly)n (1)
wherein
Hyp represents hydroxyproline, and
n represents an integer of 2 to 1000.

2. The cell culture supplement according to claim 1, which is for use in the production of a stem cell.

3. The cell culture supplement according to claim 2, wherein the stem cell is a pluripotent cell or a cancer stem cell.

4. The cell culture supplement according to claim 1, which is for use in the formation of a spheroid.

5. A spheroid-forming agent comprising a polypeptide represented by the following formula (1):
(Pro-Hyp-Gly)n (1)
wherein
Hyp represents hydroxyproline, and
n represents an integer of 2 to 1000.

6. A cell culture substrate having a culture surface treated with the cell culture supplement of any one of claims 1 to 4 or the spheroid-forming agent of claim 5.

7. A cell culture system comprising
the cell culture supplement of any one of claims 1 to 4 or the spheroid-forming agent of claim 5, and
a cultured cell.

8. A method for producing a stem cell, comprising
culturing a cell by using the cell culture supplement of any one claims 1 to 4 or the spheroid-forming agent of claim 5.

9. The method according to claim 8, wherein the stem cell expresses at least one undifferentiated stem cell marker gene selected from the group consisting of SOX2, OCT4, and NANOG.

10. The method according to claim 8, wherein the stem cell is a pluripotent stem cell or a cancer stem cell.

11. A method for producing a tissue, using a stem cell obtained by the method of claim 8.
